(19) Europäisches Patentamt
European Patent Office
Office européen des brevets

(11) EP 2 916 866 B1

## (12) EUROPÄISCHE PATENTSCHRIFT

(45) Veröffentlichungstag und Bekanntmachung des Hinweises auf die Patenterteilung:
**04.04.2018 Patentblatt 2018/14**

(21) Anmeldenummer: **13786255.3**

(22) Anmeldetag: **05.11.2013**

(51) Int Cl.:
*A61K 39/395* [(2006.01)]     *C07K 16/28* [(2006.01)]
*C07K 16/30* [(2006.01)]     *A61P 35/00* [(2006.01)]

(86) Internationale Anmeldenummer:
**PCT/EP2013/073024**

(87) Internationale Veröffentlichungsnummer:
**WO 2014/072277 (15.05.2014 Gazette 2014/20)**

(54) **FORMULIERUNG FÜR BISPECIFIC T-CELL-ENGANGERS (BITES)**

FORMULATION FOR BISPECIFIC T-CELL ENGAGERS (BITES)

FORMULATION D'ACTIVATEURS DE LYMPHOCYTES T BISPÉCIFIQUES (BITE)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.11.2012 EP 12191493**

(43) Veröffentlichungstag der Anmeldung:
**16.09.2015 Patentblatt 2015/38**

(73) Patentinhaber: **Bayer Pharma Aktiengesellschaft 13353 Berlin (DE)**

(72) Erfinder:
- **OLBRICH, Carsten**
  **10961 Berlin (DE)**
- **BUNTE, Thomas**
  **12307 Berlin (DE)**
- **WINTER, Jonas**
  **94920 Tiburon (US)**
- **PETERS, Jörg**
  **40699 Erkrath (DE)**
- **TRILL, Thomas**
  **16552 Schildow (DE)**

(74) Vertreter: **BIP Patents
c/o Bayer Intellectual Property GmbH
Alfred-Nobel-Straße 10
40789 Monheim am Rhein (DE)**

(56) Entgegenhaltungen:
**WO-A1-2009/070642     WO-A2-2010/037836**

- **CHOI BRYAN D ET AL: "Bispecific antibodies engage T cells for antitumor immunotherapy", EXPERT OPINION ON BIOLOGICAL THERAPY, INFORMA HEALTHCARE, UK, Bd. 11, Nr. 7, 1. Juli 2011 (2011-07-01), Seiten 843-853, XP009169599, ISSN: 1744-7682, DOI: 10.1517/14712598.2011.572874**
- **TANAKA K ET AL: "CRYOPROTECTIVE EFFECT OF SACCHARIDES ON DENATURATION OF CATALASE BY FREEZE-DRYING", CHEMICAL AND PHARMACEUTICAL BULLETIN (TOKYO), vol. 39, no. 5, 1991, pages 1091-1094, ISSN: 0009-2363**
- **LIAO YONG-HONG ET AL: "Protective mechanism of stabilizing excipients against dehydration in the freeze-drying of proteins", PHARMACEUTICAL RESEARCH, SPRINGER NEW YORK LLC, US, vol. 19, no. 12, 1 December 2002 (2002-12-01), pages 1854-1861, XP002377187, ISSN: 0724-8741, DOI: 10.1023/A:1021497625645**
- **CONSTANTINO H R ET AL: "EFFECT OF EXCIPIENTS ON THE STABILITY AND STRUCTURE OF LYOPHILIZED RECOMBINANT HUMAN GROWTH HORMONE", JOURNAL OF PHARMACEUTICAL SCIENCES, AMERICAN PHARMACEUTICAL ASSOCIATION, WASHINGTON, US, vol. 87, no. 11, 1 November 1998 (1998-11-01), pages 1412-1420, XP000783391, ISSN: 0022-3549, DOI: 10.1021/JS980069T**

SEQUENCE LISTING

<110> Bayer Pharma AG

<120> Formulierung für Polypeptide

<130> BHC 11 1 057

<160> 8

<170> PatentIn version 3.5

<210> 1
<211> 27
<212> PRT
<213> Homo sapiens

<400> 1

```
        Gln Asp Gly Asn Glu Glu Met Gly Gly Ile Thr Gln Thr Pro Tyr Lys
        1               5                   10                  15

        Val Ser Ile Ser Gly Thr Thr Val Ile Leu Thr
                    20                  25
```

<210> 2
<211> 27
<212> PRT
<213> Callithrix jacchus

<400> 2

```
        Gln Asp Gly Asn Glu Glu Met Gly Asp Thr Thr Gln Asn Pro Tyr Lys
        1               5                   10                  15

        Val Ser Ile Ser Gly Thr Thr Val Thr Leu Thr
                    20                  25
```

<210> 3
<211> 27
<212> PRT
<213> Saguinus oedipus

<400> 3

```
        Gln Asp Gly Asn Glu Glu Met Gly Asp Thr Thr Gln Asn Pro Tyr Lys
        1               5                   10                  15

        Val Ser Ile Ser Gly Thr Thr Val Thr Leu Thr
                        20                  25
```

<210> 4
<211> 27
<212> PRT
<213> Saimiri sciureus

<400> 4

```
        Gln Asp Gly Asn Glu Glu Ile Gly Asp Thr Thr Gln Asn Pro Tyr Lys
        1               5                   10                  15

        Val Ser Ile Ser Gly Thr Thr Val Thr Leu Thr
                        20                  25
```

<210> 5
<211> 249
<212> PRT
<213> Artificial Sequence

<220>
<223> scFv Bindedomäne CD3

<400> 5

```
Glu Val Gln Leu Val Glu Ser Gly Gly Gly Leu Val Gln Pro Gly Gly
1               5               10              15

Ser Leu Lys Leu Ser Cys Ala Ala Ser Gly Phe Thr Phe Asn Lys Tyr
            20              25              30

Ala Met Asn Trp Val Arg Gln Ala Pro Gly Lys Gly Leu Glu Trp Val
        35              40              45

Ala Arg Ile Arg Ser Lys Tyr Asn Asn Tyr Ala Thr Tyr Tyr Ala Asp
    50              55              60

Ser Val Lys Asp Arg Phe Thr Ile Ser Arg Asp Asp Ser Lys Asn Thr
65              70              75              80

Ala Tyr Leu Gln Met Asn Asn Leu Lys Thr Glu Asp Thr Ala Val Tyr
            85              90              95

Tyr Cys Val Arg His Gly Asn Phe Gly Asn Ser Tyr Ile Ser Tyr Trp
            100             105             110

Ala Tyr Trp Gly Gln Gly Thr Leu Val Thr Val Ser Ser Gly Gly Gly
        115             120             125

Gly Ser Gly Gly Gly Gly Ser Gly Gly Gly Gly Ser Gln Thr Val Val
    130             135             140

Thr Gln Glu Pro Ser Leu Thr Val Ser Pro Gly Gly Thr Val Thr Leu
145             150             155             160

Thr Cys Gly Ser Ser Thr Gly Ala Val Thr Ser Gly Asn Tyr Pro Asn
            165             170             175

Trp Val Gln Gln Lys Pro Gly Gln Ala Pro Arg Gly Leu Ile Gly Gly
        180             185             190

Thr Lys Phe Leu Ala Pro Gly Thr Pro Ala Arg Phe Ser Gly Ser Leu
        195             200             205

Leu Gly Gly Lys Ala Ala Leu Thr Leu Ser Gly Val Gln Pro Glu Asp
    210             215             220

Glu Ala Glu Tyr Tyr Cys Val Leu Trp Tyr Ser Asn Arg Trp Val Phe
225             230             235             240

Gly Gly Gly Thr Lys Leu Thr Val Leu
            245
```

EP 2 916 866 B1

<210> 6
<211> 243
<212> PRT
<213> Artificial Sequence

<220>
<223> scFv Bindedomäne PSMA

<400> 6

```
Gln Val Gln Leu Val Glu Ser Gly Gly Gly Leu Val Lys Pro Gly Glu
1               5               10              15

Ser Leu Arg Leu Ser Cys Ala Ala Ser Gly Phe Thr Phe Ser Asp Tyr
            20              25              30

Tyr Met Tyr Trp Val Arg Gln Ala Pro Gly Lys Gly Leu Glu Trp Val
            35              40              45

Ala Ile Ile Ser Asp Gly Gly Tyr Tyr Thr Tyr Tyr Ser Asp Ile Ile
        50              55              60

Lys Gly Arg Phe Thr Ile Ser Arg Asp Asn Ala Lys Asn Ser Leu Tyr
65              70              75              80

Leu Gln Met Asn Ser Leu Lys Ala Glu Asp Thr Ala Val Tyr Tyr Cys
                85              90              95

Ala Arg Gly Phe Pro Leu Leu Arg His Gly Ala Met Asp Tyr Trp Gly
            100             105             110

Gln Gly Thr Leu Val Thr Val Ser Ser Gly Gly Gly Gly Ser Gly Gly
            115             120             125

Gly Gly Ser Gly Gly Gly Gly Ser Asp Ile Gln Met Thr Gln Ser Pro
```

```
                130                      135                         140


        Ser Ser Leu Ser Ala Ser Val Gly Asp Arg Val Thr Ile Thr Cys Lys
        145                 150                 155                 160


        Ala Ser Gln Asn Val Asp Thr Asn Val Ala Trp Tyr Gln Gln Lys Pro
                        165                 170                 175


        Gly Gln Ala Pro Lys Ser Leu Ile Tyr Ser Ala Ser Tyr Arg Tyr Ser
                    180                 185                 190


        Asp Val Pro Ser Arg Phe Ser Gly Ser Ala Ser Gly Thr Asp Phe Thr
                    195                 200                 205


        Leu Thr Ile Ser Ser Val Gln Ser Glu Asp Phe Ala Thr Tyr Tyr Cys
            210                 215                 220


        Gln Gln Tyr Asp Ser Tyr Pro Tyr Thr Phe Gly Gly Gly Thr Lys Leu
        225                 230                 235                 240


        Glu Ile Lys
```

<210> 7
<211> 498
<212> PRT
<213> Artificial Sequence

<220>
<223> PSMA-BiTE

<400> 7

```
Gln Val Gln Leu Val Glu Ser Gly Gly Gly Leu Val Lys Pro Gly Glu
1               5               10              15

Ser Leu Arg Leu Ser Cys Ala Ala Ser Gly Phe Thr Phe Ser Asp Tyr
            20              25              30

Tyr Met Tyr Trp Val Arg Gln Ala Pro Gly Lys Gly Leu Glu Trp Val
        35              40              45

Ala Ile Ile Ser Asp Gly Gly Tyr Tyr Thr Tyr Tyr Ser Asp Ile Ile
    50              55              60

Lys Gly Arg Phe Thr Ile Ser Arg Asp Asn Ala Lys Asn Ser Leu Tyr
65              70              75              80

Leu Gln Met Asn Ser Leu Lys Ala Glu Asp Thr Ala Val Tyr Tyr Cys
            85              90              95
```

```
Ala Arg Gly Phe Pro Leu Leu Arg His Gly Ala Met Asp Tyr Trp Gly
            100             105             110

Gln Gly Thr Leu Val Thr Val Ser Ser Gly Gly Gly Ser Gly Gly
            115             120             125

Gly Gly Ser Gly Gly Gly Gly Ser Asp Ile Gln Met Thr Gln Ser Pro
            130             135             140

Ser Ser Leu Ser Ala Ser Val Gly Asp Arg Val Thr Ile Thr Cys Lys
145             150             155             160

Ala Ser Gln Asn Val Asp Thr Asn Val Ala Trp Tyr Gln Gln Lys Pro
                165             170             175

Gly Gln Ala Pro Lys Ser Leu Ile Tyr Ser Ala Ser Tyr Arg Tyr Ser
            180             185             190

Asp Val Pro Ser Arg Phe Ser Gly Ser Ala Ser Gly Thr Asp Phe Thr
            195             200             205

Leu Thr Ile Ser Ser Val Gln Ser Glu Asp Phe Ala Thr Tyr Tyr Cys
            210             215             220

Gln Gln Tyr Asp Ser Tyr Pro Tyr Thr Phe Gly Gly Gly Thr Lys Leu
225             230             235             240

Glu Ile Lys Ser Gly Gly Gly Gly Ser Glu Val Gln Leu Val Glu Ser
            245             250             255

Gly Gly Gly Leu Val Gln Pro Gly Gly Ser Leu Lys Leu Ser Cys Ala
            260             265             270

Ala Ser Gly Phe Thr Phe Asn Lys Tyr Ala Met Asn Trp Val Arg Gln
            275             280             285

Ala Pro Gly Lys Gly Leu Glu Trp Val Ala Arg Ile Arg Ser Lys Tyr
            290             295             300

Asn Asn Tyr Ala Thr Tyr Tyr Ala Asp Ser Val Lys Asp Arg Phe Thr
305             310             315             320

Ile Ser Arg Asp Asp Ser Lys Asn Thr Ala Tyr Leu Gln Met Asn Asn
            325             330             335

Leu Lys Thr Glu Asp Thr Ala Val Tyr Tyr Cys Val Arg His Gly Asn
```

34

340                        345                        350

```
Phe Gly Asn Ser Tyr Ile Ser Tyr Trp Ala Tyr Trp Gly Gln Gly Thr
        355             360             365

Leu Val Thr Val Ser Ser Gly Gly Gly Ser Gly Gly Gly Gly Ser
    370             375             380

Gly Gly Gly Gly Ser Gln Thr Val Val Thr Gln Glu Pro Ser Leu Thr
385             390             395             400

Val Ser Pro Gly Gly Thr Val Thr Leu Thr Cys Gly Ser Ser Thr Gly
                405             410             415

Ala Val Thr Ser Gly Asn Tyr Pro Asn Trp Val Gln Gln Lys Pro Gly
        420             425             430

Gln Ala Pro Arg Gly Leu Ile Gly Gly Thr Lys Phe Leu Ala Pro Gly
        435             440             445

Thr Pro Ala Arg Phe Ser Gly Ser Leu Leu Gly Gly Lys Ala Ala Leu
    450             455             460

Thr Leu Ser Gly Val Gln Pro Glu Asp Glu Ala Glu Tyr Tyr Cys Val
465             470             475             480

Leu Trp Tyr Ser Asn Arg Trp Val Phe Gly Gly Gly Thr Lys Leu Thr
                485             490             495

Val Leu
```

<210> 8
<211> 504
<212> PRT
<213> Artificial Sequence

<220>
<223> Sequenz des PSMA-BiTE1

<400> 8

Gln Val Gln Leu Val Glu Ser Gly Gly Gly Leu Val Lys Pro Gly Glu
1               5               10              15

Ser Leu Arg Leu Ser Cys Ala Ala Ser Gly Phe Thr Phe Ser Asp Tyr
            20              25              30

Tyr Met Tyr Trp Val Arg Gln Ala Pro Gly Lys Gly Leu Glu Trp Val
        35              40              45

Ala Ile Ile Ser Asp Gly Gly Tyr Tyr Thr Tyr Tyr Ser Asp Ile Ile
50          55                60

Lys Gly Arg Phe Thr Ile Ser Arg Asp Asn Ala Lys Asn Ser Leu Tyr
65          70                75                80

Leu Gln Met Asn Ser Leu Lys Ala Glu Asp Thr Ala Val Tyr Tyr Cys
85                90                95

Ala Arg Gly Phe Pro Leu Leu Arg His Gly Ala Met Asp Tyr Trp Gly
100                105                110

Gln Gly Thr Leu Val Thr Val Ser Ser Gly Gly Gly Gly Ser Gly Gly
115                120                125

Gly Gly Ser Gly Gly Gly Gly Ser Asp Ile Gln Met Thr Gln Ser Pro
130                135                140

Ser Ser Leu Ser Ala Ser Val Gly Asp Arg Val Thr Ile Thr Cys Lys
145                150                155                160

Ala Ser Gln Asn Val Asp Thr Asn Val Ala Trp Tyr Gln Gln Lys Pro
165                170                175

Gly Gln Ala Pro Lys Ser Leu Ile Tyr Ser Ala Ser Tyr Arg Tyr Ser
180                185                190

Asp Val Pro Ser Arg Phe Ser Gly Ser Ala Ser Gly Thr Asp Phe Thr
195                200                205

Leu Thr Ile Ser Ser Val Gln Ser Glu Asp Phe Ala Thr Tyr Tyr Cys
210                215                220

Gln Gln Tyr Asp Ser Tyr Pro Tyr Thr Phe Gly Gly Gly Thr Lys Leu
225                230                235                240

Glu Ile Lys Ser Gly Gly Gly Gly Ser Glu Val Gln Leu Val Glu Ser
245                250                255

Gly Gly Gly Leu Val Gln Pro Gly Gly Ser Leu Lys Leu Ser Cys Ala
260                265                270

Ala Ser Gly Phe Thr Phe Asn Lys Tyr Ala Met Asn Trp Val Arg Gln
275                280                285

Ala Pro Gly Lys Gly Leu Glu Trp Val Ala Arg Ile Arg Ser Lys Tyr

290                    295                    300

Asn Asn Tyr Ala Thr Tyr Tyr Ala Asp Ser Val Lys Asp Arg Phe Thr
305                     310                     315                     320

Ile Ser Arg Asp Asp Ser Lys Asn Thr Ala Tyr Leu Gln Met Asn Asn
                    325                     330                     335

Leu Lys Thr Glu Asp Thr Ala Val Tyr Tyr Cys Val Arg His Gly Asn
                    340                     345                     350

Phe Gly Asn Ser Tyr Ile Ser Tyr Trp Ala Tyr Trp Gly Gln Gly Thr
                    355                     360                     365

Leu Val Thr Val Ser Ser Gly Gly Gly Gly Ser Gly Gly Gly Gly Ser
                    370                     375                     380

Gly Gly Gly Gly Ser Gln Thr Val Val Thr Gln Glu Pro Ser Leu Thr
385                     390                     395                     400

Val Ser Pro Gly Gly Thr Val Thr Leu Thr Cys Gly Ser Ser Thr Gly
                    405                     410                     415

Ala Val Thr Ser Gly Asn Tyr Pro Asn Trp Val Gln Gln Lys Pro Gly
                    420                     425                     430

Gln Ala Pro Arg Gly Leu Ile Gly Gly Thr Lys Phe Leu Ala Pro Gly
                    435                     440                     445

Thr Pro Ala Arg Phe Ser Gly Ser Leu Leu Gly Gly Lys Ala Ala Leu
                    450                     455                     460

Thr Leu Ser Gly Val Gln Pro Glu Asp Glu Ala Glu Tyr Tyr Cys Val
465                     470                     475                     480

Leu Trp Tyr Ser Asn Arg Trp Val Phe Gly Gly Gly Thr Lys Leu Thr
                    485                     490                     495

Val Leu His His His His His His
                    500

**Beschreibung**

Einleitung

Die vorliegende Erfindung bezieht sich auf stabile pharmazeutische Zusammensetzungen, die Polypeptide mit mindestens zwei Antigen-Bindedomänenenthalten und besonders geeignet sind für die subkutane Applikation. Die Erfindung stellt flüssige Zusammensetzungen bereit, welche die Bildung unerwünschter Polypeptid-Aggregate (Dimere und/oder Multimere) minimieren. Die vorliegende Erfindung stellt weiterhin eine Methode zur Minimierung der Aggregation von Polypeptiden mit Antigen-Bindedomänen in flüssigen Zusammensetzungen bereit.

Stand der Technik

Die Notwendigkeit, Antikörper in Lösung zu stabilisieren, d.h. die Bildung von Dimeren und Multimeren (sog. High Molecular Weight oder HMW-Aggregaten) zu verhindern, um die therapeutische Wirksamkeit konstant zu halten, ist im Stand der Technik bekannt. WO2011061712 offenbart stabilisierte Antikörper-Formulierungen, die neben 25 - 250 mg/ml Antikörper 10-30 mM eines Puffers (bevorzugt Acetat, Succinat, Phosphat, Histidin oder Kombinationen hiervon), 1-15% eines Polyols sowie 0,001-0,05% eines Netzmittels enthalten. Der pH-Wert der Zusammensetzungen liegt zwischen 5-7,5.

WO2010148337(A1) ("Lyophilized formulation for small modular immunopharmaceuticals") offenbart Zusammensetzungen so genannter Small Immunopharmaceutical Proteins (SMIP). Dies sind Konstrukte aus mehreren, fusionierten Domänen, wie z.B. einer Antigen-Bindedomäne, einer Immunglobulin-Gelenkregion und einer $C_H2$- oder $C_H3$-Region eines Ig-Moleküls oder einer hiervon abgeleiteten Region. Die Domänen der SMIPs bestehen aus Polypeptiden, die Produkte von Gensequenzen sind, die humaner, nicht-humaner oder artifizieller (mit Hilfe gentechnischer Methoden erzeugter) Herkunft sein können. SMIP-Proteine sind zwar bevorzugt monospezifisch, in der Anmeldung sind aber auch multispezifische Varianten offenbart, wie beispielsweise Scorpion-Moleküle. Diese enthalten SMIP-Proteine mit einer weiteren, C-terminalen Bindedomäne. Die Bindedomänen der Scorpion-Moleküle binden bevorzugt an unterschiedliche Zielstrukturen und eigenen sich daher als immunspezifische Therapeutika. WO2010148337(A1) offenbart stabile Formulierungen lyophylisierte Zusammensetzungen enthaltend ein SMIP, wobei weniger als 7% des SMIP in aggregierter Form vorliegt. Diese Formulierungen können weiterhin Pufferagentien, Stabilisatoren, Bulking Agentien, Netzmittel und weitere Hilfsstoffe enthalten. WO2009070642 A1 offenbart verschiedene Formulierungen des BiTE-Moleküls MT103, dessen erste Bindedomäne spezifisch an das T-Zellrezeptor-Antigen CD3 bindet, während die zweite spezifisch an das B-Zell-Antigen CD19 bindet. Die BiTE-Moleküle sind in den offenbarten Zusammensetzungen stabil bis zu einer Konzentration von maximal 300 $\mu$g/mL, bei einem pH-Wert von 7,0. Als Puffer wird Citrat eingesetzt. Die Formulierungen eignen sich zur intravenösen und subkutanen Applikation. Die Bioverfügbarkeit nach subkutaner Applikation liegt bei 10-50%.

IgG-Antikörper besitzen große konstante Bereiche ($C_{H1-3}$-/ $C_L$-Regionen), die für einen Großteil ihrer physikochemischen Eigenschaften verantwortlich sind. IgG-Antikörper mit unterschiedlicher Spezifität unterscheiden sich strukturell hauptsächlich im Bereich der hypervariablen Antikörperbindungsstellen (CDR1-CDR3) innerhalb der $V_L$- und $V_H$-Regionen. Die strukturellen und physikochemischen Unterschiede zwischen den einzelnen IgG-Varianten sind aufgrund der großen konstanten Bereiche relativ gering. Wie IgG-Antikörper enthalten die in WO2010148337 (A1) beschriebenen SMIP Moleküle Teile der konstanten Antikörperdomäne.

Dagegen unterscheiden sich BiTE-Moleküle mit unterschiedlicher Spezifität deutlich in ihren physikochemischen Eigenschaften. Als Fusionsprotein aus zwei single chain variablen Fragmenten (scFv) idR. unterschiedlicher Immunglobuline fehlen ihnen die konstanten $C_{H1-3}$-/ $C_L$-Regionen, so dass Unterschiede in den Antigen-Bindedomänen weitaus größere Teilbereiche der BiTE-Moleküle betreffen, als dies bei IgG-/oder SMIP Antikörpern der Fall ist. Desgleichen unterscheiden sich das BiTE Molekül MT103 und die BiTE Moleküle, die in der vorliegenden Erfindung verwendet werden, grundsätzlich in ihrer molekularen Struktur. Während die Domänen bei MT103 in der Reihenfolge $V_L$-$V_H$-$V_H$-$V_L$ angeordnet sind, ist die Anordnung bei BiTE Moleküle, die in der vorliegenden Erfindung bevorzugt verwendet werden in der Form $V_H$-$V_L$-$V_H$-$V_L$ vor. Darüber hinaus unterscheiden sich die Sequenzen beider Moleküle an zahlreichen Positionen.

Diese Eigenschaften der BiTE-Moleküle sowie ihre geringe Größe bedingen deutliche Unterschiede im physikochemischen Verhalten unterschiedlicher BiTE-Moleküle. Daraus ergibt sich die Notwendigkeit, individuelle Formulierungen (zur Erhöhung der physikochemischen Stabilitäten) für jede einzelne Anwendung zu entwickeln, da die Formulierungen einzelner BiTEs oder ähnlicher Moleküle nicht oder nur eingeschränkt für alternative Anwendungen verwendet werden können.

In einer Ausführungsform sind die in den Zusammensetzungen enthaltenen Polypeptide sogenannte *Bispecific T-cell-engangers* (BiTEs). In einer spezifischen Ausführungsform besitzen BiTEs eine erste Bindedomäne, die spezifisch an die ε-Kette des T-Zellrezeptor-CD3-Komplexes bindet und eine zweite Bindedomäne, die spezifisch an das Prostata-spezifische Membranantigen (PSMA) bindet. PSMA ist ein integrales Typ-II-Membranprotein, das hochspezifisch und bei Prostatakrebs verstärkt auf Prostata-Epithelzellen exprimiert wird. PSMA wird darüber hinaus von neu gebildeten Blutgefäßen solider Tumoren exprimiert. PSMA-BiTEs vermitteln somit einen direkten Kontakt zwischen cytotoxischen T-Zellen und diesen Zielzellen.

Aggregatbildung von Proteinen, wie z.B. BiTE-Molekülen, ist bei pharmazeutischen Anwendungen unerwünscht, z.B. kann die Wirksamkeit oder Verfügbarkeit eines biologischen Wirkstoffes durch Aggregatbildung verändert werden.

Daraus ergibt sich die Aufgabe eine Formulierung bereitzustellen, die es erlaubt, BiTE-Moleküle so zu stabilisieren, dass eine unerwünschte Aggregatbildung unterdrückt wird.

Die Lösung ist in der vorliegenden Anmeldung und in den Ansprüchen dargelegt und umfasst eine BiTE-Formulierung umfassend TRIS und Phosphat. In ihrer bevorzugten Ausführungsform umfasst die Formulierung 50 mM Phosphat, 100 mM TRIS, 0,04% Polysorbat 80 und 4% Trehalose bei einem pH-Wert von 6,0 und ist in der Lage, Formulierungen mit PSMA-BiTE1-Molekülen gegenüber der Bildung von Aggregaten zu stabilisieren. Dies gilt für niedrige Konzentrationen im Bereich weniger $\mu$g/ml ebenso wie für hohe Konzentrationen von >2mg/ml. Der stabilisierende Effekt ist für den Fachmann überraschend, da z.B. das in WO2009070642 A1 verwendete Citrat, auch als Kombination aus 50 mM Citrat und 100 mM TRIS bei pH 6,0, diesen Effekt nicht zeigt. So lag der gemessene Dimeranteil bei einer vergleichbaren Zusammensetzung, die lediglich Citrat statt Phosphat enthielt, bei 7,0%. Die erfindungsgemäße Zusammensetzung begrenzte den Dimeranteil dagegen auf 0,8% (vgl. Tabelle 6 und 7). Verantwortlich für den stabilisierenden Effekt der Zusammensetzungen ist der kombinierte Einsatz von TRIS und Phosphat. Um die BiTE-Moleküle auch gegenüber Scherkräften zu stabilisieren, ist ein Netzmittel wie Polysorbat 80 in einer Konzentration von mindestens 0,04 % erforderlich, da sonst der Dimeranteil zu groß wird (ca. 7,5%, siehe Tabelle 15). Um die Adsorption der PSMA-BiTE1-Moleküle an der Gefäßwandung von Injektionsspritzen, Infusionsbeuteln, etc. zu verhindern, reichen bereits 0,002% Polysorbat 80 aus.

Definitionen

Der hierin verwendete Begriff "Antikörper" bezieht sich auf Immunglobulinmoleküle, die je zwei schwere (H) und zwei leichte (L) Polypeptidketten umfassen, die über Disulfidbrücken miteinander verbunden sind. Jede schwere Kette besteht aus einer variablen Region ($V_H$) und einer konstanten Region, die wiederum aus drei Domänen ($C_H1$, $C_H2$ und $C_H3$) besteht. Jede der leichten Ketten setzt sich aus einer variablen Region ($V_L$) und eine konstanten Region ($C_L$) zusammen. Die variablen Regionen sowohl der leichten als auch der schweren Ketten ($V_H$ und $V_L$) sind weiter unterteilt, in je drei hypervariable Antikörperbindungsstellen (CDR1-CDR3) und insgesamt vier konservierte Bereiche zwischen den CDRs (FR1-FR4).

Der Begriff "monoklonaler Antikörper" bezeichnet einen Antikörper, der aus einer Population von Antikörpern stammt, die bis auf kleinere, natürlich auftretende Mutationen oder post-translationale Modifikationen identisch sind. Im Gegensatz zu polyklonalen Antikörpern, wie sie im Rahmen der Immunantwort auftreten, sind monoklonale Antikörper gegen ein spezifisches Epitop gerichtet.

Ein "bi-spezifischer" oder "bi-funktionaler Antikörper" ist ein artifizieller, hybrider Antikörper mit zwei unterschiedlichen Paaren aus schwerer und leichter Kette sowie zwei unterschiedlichen Antigen-Bindungsstellen.

Eine Behandlung von Antikörpern mit Papain führt zu zwei identischen, Antigen-bindenden Fab-Fragmenten sowie dem kristallisierbaren Fc-Fragment. Ein "Fab-Fragment" besteht aus einer kompletten $V_L$-Kette und einem Teil der schweren Kette, nämlich der $V_H$-Domäne mit der variablen Region und der ersten konstanten Domäne $C_H1$. Jedes Fab-Fragment besitzt damit eine einzelne Antigen-Bindungsstelle. Das "Fc-Fragment" umfasst die carboxy-terminalen Teile beider schweren Ketten, verknüpft über Disulfidbrücken. Teile des Fc-Fragments werden von Fc-Rezeptoren anderer Zellen erkannt und bestimmen hierüber die Effektorfunktionen der Antikörper.

Pepsin spaltet Antikörper unterhalb der Disulfidbrücken, so dass die beiden Fab-Fragmente über die Gelenkregion verbunden bleiben und ein einzelnes "F(ab')$_2$-Fragment" entsteht. Dieses verfügt über beide Antigen-Bindungsstellen und ist daher wie der vollständige Antikörper in der Lage, Antigene zu vernetzen.

Der Begriff "Domäne" bezeichnet eine globuläre Region eines Proteins mit einer definierten und unabhängig gefalteten Struktur. Die leichten Ketten eines IgG-Antikörpers setzen sich aus zwei Domänen zusammen (je eine konstante und eine variable), die schweren Ketten aus vier Domänen (je drei konstante und eine variable). Die beiden variablen Regionen setzen sich aus je einer Domäne der schweren und einer Domäne der leichten Kette zusammen.

Der Begriff "Epitop" oder "Antigene Determinante" bezeichnet den Bereich eines Antigens, an den ein Antikörper (oder das Antigen-bindende Fragment hiervon) spezifisch bindet. Epitope können aus aufeinanderfolgenden Aminosäuren bestehen, oder aus nicht aufeinanderfolgenden, deren räumliche Nähe zueinander durch tertiäre Proteinfaltung zustande kommt.

Ein "Antigen" ist ein Molekül (z.B. ein Protein, Polypeptid, Peptid, Kohlenhydrat) mit einer "antigenen Determinante", an die ein Antikörper binden kann.

Der Begriff "Konformation" bezieht sich auf die Tertiärstruktur eines Proteins oder Polypeptids, z.B. eines Antikörpers, einer Antikörperlette, einer Domäne oder eines Teils hiervon.

Ein Antikörper, der ein bestimmtes Polypeptid oder ein Epitop auf einem bestimmten Polypeptid "spezifisch bindet", oder "spezifisch für" diese Struktur ist, bindet an alternative Strukturen erheblich weniger effektiv.

Der Begriff "scFv-Antikörper" in dieser Anmeldung bezieht sich auf künstlich hergestellte Antikörperfragmente, bestehend aus kovalent gebundenen $V_H$ und $V_L$ Domänen eines Antikörpers. Beide Domänen liegen in einer einzigen Polypeptidkette vor und sind über einen Polypeptid-Linker aus mehreren Aminosäuren miteinander verbunden. Bis auf die Fc-vermittelten Effektorfunktionen bleiben bei scFv-Antikörpern alle Funktionen eines Antikörpers erhalten, insbesondere seine Selektivität und Affinität.

"Bispecific T-cell-enganger" (BiTE)-Moleküle sind rekombinante Proteinkonstrukte aus zwei flexibel verbundenen Einzelketten-Antikörpern (scFv). Einer dieser scFv-Antikörper bindet spezifisch an ein ausgewähltes, durch die Zielzellen exprimiertes Tumorantigen, der zweite bindet spezifisch an CD3, eine Untereinheit des T-Zell-Rezeptorkomplexes auf T-Zellen. Die BiTE-Antikörper sind in der Lage, T-Zellen transient an Zielzellen zu binden und gleichzeitig die cytolytische Aktivität der T-Zellen zu aktivieren. Für die BiTE-vermittelte Aktivierung der T-Zellen sind weder spezifische T-Zellrezeptoren auf den T-Zellen erforderlich, noch MHC-I-Moleküle, Peptidantigene oder co-stimulatorische Moleküle auf der Zielzelle.

Die Begriffe "Stabilität" und "stabil" im Kontext von und von BiTE-Molekülen enthaltenden Verbindungen bezeichnen die Resistenz der Antikörper bzw. ihrer Fragmente gegenüber Aggregation, Degradation oder Fragmentierung unter den zu ihrer Herstellung, Zubereitung, Lagerung, Anwendung oder ihrem Transport gegebenen Bedingungen. "Stabile" Formulierungen gemäß der vorliegenden Erfindung behalten ihre biologische Aktivität unter den gegebenen Herstellungs-, Zubereitungs-, Transport-, Anwendungs- und Lagerungsbedingungen.

In Lösung befindliche Proteine (z.B. BiTE-Moleküle) sind empfindlich gegenüber mechanischer Bewegung, wie sie während Herstellung, Abfüllung und Transport auftritt. Ab einer gewissen Bewegungsintensität aggregieren und/oder denaturieren die Moleküle. Flüssige, proteinhaltige Zusammensetzungen sind damit während mechanischer Bewegung einem sog. Schüttelstress ausgesetzt. Beim "Schüttel-Stresstest" wird durch den kontrollierten Einsatz mechanischer (Schüttel-) Kräfte auf flüssige, proteinhaltige Zusammensetzungen das Aggregations- und Denaturierungsverhalten des gelösten Proteins in unterschiedlichen Zusammensetzungen analysiert.

Das Verhalten eines Proteins im Schüttel-Stresstest ist ein Hinweis auf dessen physikalische Stabilität gegenüber Scherkräften, wie sie z.B. bei der Aspiration und Injektion von Lösungen mit Kanülen oder auftreten.

"Lyophilisation" bezeichnet ein Trocknungsverfahren, das auf dem Prinzip der Sublimation beruht. Die zu trocknende Substanz wird zunächst auf etwa -45°C heruntergekühlt, bevor anschließend ein Vakuum angelegt und die Substanz auf etwa -20°C erwärmt wird. Hierdurch sublimieren die Eiskristalle direkt in den gasförmigen Zustand, ohne dass ein flüssiger Zwischenschritt durchlaufen wird. Die auf diese Weise getrocknete Substanz enthält nach einem Nachtrocknungsschritt (immer noch unter Vakuum) bei etwa 25°C weniger als 5% ihrer ursprünglichen Feuchtigkeit und wird als "Lyophilisat" bezeichnet.

Vor der Verabreichung an den Patienten wird das Lyophilisat "rekonstituiert", d.h. in einem pharmazeutisch akzeptablen Verdünnungsmittel gelöst. Eine "rekonstituierte Formulierung" im Sinne der vorliegenden Erfindung entsteht durch die Lösung einer lyophilisierten Antikörper-Formulierung in einem solchen Verdünnungsmittel. Der Antikörper liegt anschließend gelöst vor und kann dem Patienten verabreicht werden.

Als "Polyole" wird eine Gruppe organischer Verbindungen bezeichnet, die mehrere Hydroxygruppen (-OH) enthalten (Polyalkohol, mehrwertiger Alkohol). Polyole wie Sucrose oder Trehalose sind reduzierende Zucker, die in der Lage sind, Antikörper zu stabilisieren und/oder die Osmolarität einer Zusammensetzung zu beeinflussen.

Um eine unerwünschte Degradierung oder Aggregation von Proteinen während der Lyophilisierung zu verhindern, werden sog. "Lyoprotektiva" hinzugefügt. Hierbei handelt es sich beispielsweise um Zucker oder Zuckeralkohole wie Sucrose, Mannose, Trehalose, Glucose, Sorbitol, Mannitol. Im Kontext der vorliegenden Erfindung ist Trehalose das bevorzugt verwendete Lyoprotektivum.

Der Begriff "Netzmittel" hierin bezieht sich auf jedes Detergenz mit einer hydrophilen und einer hydrophoben Region und schließt nicht-ionische, kationische, anionische und zwitterionische Detergentien mit ein. Verwendbare Detergentien umfasse beispielsweise Polyoxyethylen-sorbitanmonooleat (auch bekannt als Polysorbat 80 oder TWEEN 80), Polyoxyethylen-sorbitan-monolaurat (auch bekannt als Polysorbat 20 oder TWEEN 20), oder N-Laurylsarcosin. Für die hierin offenbarten Zusammensetzungen ist ein nicht-ionisches Netzmittel zu bevorzugen. Besonders bevorzugt für die Zusammensetzungen der vorliegenden Erfindung ist die Verwendung von Polysorbat 80. Das Netzmittel kann verwendet werden in einer Konzentration von 0,002 % bis 0,1 %.

Der Begriff "Puffer" bezeichnet hierin eine gepufferte Lösung, deren pH-Wert sich nach Zugabe von sauren oder alkalischen Stoffen nur wenig ändert. Gepufferte Lösungen enthalten eine Mischung aus einer schwachen Säure und ihrer korrespondierenden Base oder einer schwachen Base und ihrer korrespondierenden Säure.

Der Begriff "Patient" bezieht sich auf menschliche oder tierische) Individuen, die eine vorbeugende oder therapeutische Behandlung erhalten.

Der Begriff "Behandlung" hierin bezieht sich auf die Anwendung oder Gabe eines therapeutischen Stoffes an einen Patienten, oder auf die Anwendung oder Gabe eines therapeutischen Stoffes an ein isoliertes Gewebe oder an eine Zelllinie eines Patienten, der eine Krankheit leidet, ein Symptom einer Krankheit zeigt, oder eine Prädisposition für eine Krankheit hat, mit dem Ziel, die Krankheit, ihre Symptome oder die Prädisposition für die Krankheit zu heilen, zu verbessern, zu beeinflussen, abzustellen oder zu lindern.

Als "Effektive Dosis" wird hierin die Wirkstoffmenge bezeichnet, mit der sich zumindest teilweise der gewünschte Effekt erzielen lässt. Eine "therapeutisch effektive Dosis" ist demnach definiert als die Wirkstoffmenge, die ausreicht, um eine Krankheit zumindest teilweise zu heilen, oder durch sie verursachten Beeinträchtigungen des Patienten zumindest teilweise zu beheben. Die hierfür tatsächlich erforderlichen Mengen sind abhängig vom Schweregrad der Erkrankung sowie dem allgemeinen Immunstatus des Patienten.

Der Begriff "Bioverfügbarkeit", wie hier verwendet, bezeichnet den prozentualen Anteil eines Wirkstoffes oder einer Arzneimitteldosis, die unverändert im systemischen Kreislauf zur Verfügung steht. Die Bioverfügbarkeit ist damit eine Messgröße dafür, wie schnell und in welchem Umfang der Wirkstoff resorbiert wird und am Wirkort zur Verfügung steht. Intravenös verabreichte Arzneimittel haben definitionsgemäß eine Bioverfügbarkeit von 100%.

Die absolute Bioverfügbarkeit ist die Bioverfügbarkeit einer auf beliebige (nicht-intravenöse) Weise verabreichten Substanz im Vergleich zur intravenösen Gabe bezeichnet, während sich die relative Bioverfügbarkeit aus dem Vergleich der Bioverfügbarkeiten bei einzelnen Darreichungsformen (z.B. oral vs. subkutan) ergibt.

Eine "isotonische Verbindung" besitzt im Wesentlichen denselben osmotischen Druck wie menschliches Blut. Isotonische Verbindungen haben daher im Allgemeinen einen osmotischen Druck von etwa 250 bis 350 mOsm. Der Begriff "hypotonisch" beschreibt Zusammensetzungen mit einem osmotischen Druck unterhalb dessen von menschlichem Blut, während "hypertonische" Zusammensetzungen einen osmotischen Druck oberhalb dessen von menschlichem Blut aufweisen.

Der Begriff "Hochmolekulare Aggregate" (synonym: "High Molecular Weight Aggregates", oder "HMW") bezeichnet Aggregate, die sich aus mindestens zwei Proteinmonomeren zusammensetzen.

Der hierin verwendete Begriff "Phosphate" bezieht sich auf wasserlösliche, pharmakologisch unbedenkliche Salze der dreibasigen ortho-Phosphorsäure ($H_3PO_4$), wobei primäre (Hydrogen-) und sekundäre (Dihydrogen-) Phosphate bevorzugt sind. Bevorzugt enthalten die erfindungsgemäßen Zusammensetzungen Natriumphosphate, besonders bevorzugt Dinatriumhydrogenphosphat ($Na_2HPO_4$).

Detaillierte Beschreibung

Die Erfindung betrifft die pharmazeutische Formulierung eines Bispecific T-cell-engager (BiTE) Moleküls, dadurch gekennzeichnet, dass sie Tris(hydroxymethyl)-aminomethan (TRIS) und Phosphat umfasst.

BiTE Moleküle sind im Stand der Technik bekannt. BiTE Moleküle sind so gestaltet, dass sie transient zytotoxische T-Zellen für die Lyse bestimmter Targetzellen beteiligen (siehe Bäuerle et al. Curr Opin Mol Ther. 2009 Feb;11(1):22-30.). Sie sind besonders für die Krebstherapie geeignet.

Ein BiTE Molekül ist ein Polypeptid, welches zwei scFv Antikörperbindedomänen umfasst, wobei die erste scFv Bindedomäne an das humane CD3 epsilon binden kann und die zweite scFv Bindedomäne bindet ein weiteres zweites Oberflächenantigen. Bevorzugt sind humane Oberflächenantigene von Krebszellen. Besonders bevorzugte Oberflächenantigene sind humane Oberflächenproteine von Krebszellen. Die scFv Bindedomänen können chimäre, humanisierte oder humane Antikörperfragmente umfassen. Bevorzugt umfassen die scFv Bindedomänen humane oder humanisierte Antikörperfragmente.

Die in der vorliegenden Erfindung verwendeten BiTE Moleküle unterscheiden sich von den BiTE Molekülen (z.B. MT103) wie sie z.B. in WO2009070642 A1 beschrieben sind, dadurch, dass die erste Bindedomäne an ein Epitop der humanen und Callithrix jacchus, Saguinus oedipus oder Saimiri sciureus CD3 epsilon Kette binden kann, wobei das Epitop Teil einer Aminosäuresequenz ist, die ausgewählt ist aus der Gruppe bestehend aus SEQ ID NO: 1, 2, 3 und 4 und wobei das Epitop mindestens die Aminosäuresequenz Gln-Asp-Gly-Asn-Glu umfasst. Dies hat den Vorteil, dass präklinische Untersuchungen erleichtert sind, da z.B. pharmakokinetische oder toxikologische Studien in den genannten Versuchstieren durchgeführt werden können, deren Immunsystem dem des Menschen ähnlich ist. BiTE Moleküle mit diesen Charakteristika sind in z.B in WO2008119566 A2 oder WO2008119567 A2 offenbart.

In einer Ausführungsform umfasst die erfindungsgemäße Zusammensetzung also solche BiTE Moleküle, deren erste Bindedomäne an ein Epitop der humanen und Callithrix jacchus, Saguinus oedipus oder Saimiri sciureus CD3 epsilon Kette binden kann, wobei das Epitop Teil einer Aminosäuresequenz ist, die ausgewählt ist aus der Gruppe bestehend aus SEQ ID NO: 1, 2, 3 und 4 und wobei das Epitop mindestens die Aminosäuresequenz Gln-Asp-Gly-Asn-Glu umfasst.

In SEQ ID NO: 5 ist die Aminosäuresequenz einer scFV Bindedomäne wiedergegeben, die die vorangegangen Kriterien erfüllt.

In einer bevorzugten Ausführungsform umfasst die erste Bindedomäne des Polypeptides die Aminosäuresequenz wiedergegeben in SEQ ID NO: 5.

scFV umfassen die Aminosäuren einer variablen leichten (VL) und einer variablen schweren (VH) Antikörperkette. BITE Moleküle können in unterschiedlicher Orientierung aufgebaut werden. Das BiTE Molekül MT103 hat z.B. eine (VL-VH) Bindedomäne2-(VH-VL)Bindedomäne1 Anordnung der scFVs.

Es sind auch andere Orientierungen möglich, z.B. (VH-VL)Bindedomäne2-(VH-VL)Bindedomäne1.

In einer bevorzugten Ausführungsform hat das Polypeptid die Anordnung (VH-VL)Bindedomäne2-(VH - VL)Bindedomäne1.

In einer besonders bevorzugten Ausführungsform hat das Polypeptid die Anordnung (VH-VL)Bindedomäne2-(VH-VL)Bindedomäne1, wobei (VH-VL)Bindedomäne1 die Aminosäuresequenz wiedergegeben in SEQ ID NO: 5 umfasst.

Eine Ausführungsform der vorliegenden Erfindung ist eine flüssige pharmazeutische Zusammensetzung dadurch gekennzeichnet, dass die zweite Bindedomäne an ein Zelloberflächenantigen binden kann. Ein Zelloberflächenantigen ist ein Antigen, welches von einem Bindeprotein, z.B. einem Antikörper oder einem scFv, gebunden werden kann, ohne dass die Zelle lysiert werden muss.

Eine Ausführungsform der vorliegenden Erfindung ist eine flüssige pharmazeutische Zusammensetzung, dadurch gekennzeichnet, dass die zweite Bindedomäne des Polypeptides an ein Oberflächenantigen einer Krebszelle binden kann.

In einer weiteren Ausführungsform bindet die zweite Bindedomäne des Polypeptides an das humane Oberflächenantigen Prostate-Specific Membrane Antigen (PSMA, SWISS-PROT: FOLH1_HUMAN, accession no: Q04609). Solche BiTE-Moleküle sind z.B. in WO2010037836 A2 beschrieben.

In SEQ ID NO: 6 ist eine Bindedomäne beschrieben, die an PSMA bindet.

In einer bevorzugten Ausführungsform umfasst die zweite Bindedomäne des Polypeptides die Aminosäuresequenz wiedergegeben in SEQ ID NO: 6

Eine Ausführungsform der vorliegenden Erfindung ist eine flüssige pharmazeutische Zusammensetzung, umfassend ein Polypeptid, welches eine erste und eine zweite scFv Bindedomäne umfasst, wobei die erste Bindedomäne die Aminosäuresequenz wiedergegeben in SEQ ID NO: 5 umfasst, dadurch gekennzeichnet, dass die Zusammensetzung weiterhin TRIS und Phosphat umfasst.

Eine Ausführungsform der vorliegenden Erfindung ist eine flüssige pharmazeutische Zusammensetzung dadurch gekennzeichnet, dass die Bindedomänen des Polypeptides humane oder humanisierte scFv Antikörperfragmente umfassen.

Eine Ausführungsform der vorliegenden Erfindung ist eine flüssige pharmazeutische Zusammensetzung dadurch gekennzeichnet, dass die zweite PSMA-bindende Bindedomäne die Aminosäuresequenz wiedergegeben in SEQ ID NO: 6 umfasst.

In einer Ausführungsform umfasst das Polypeptid die Aminosäuresequenzen der ersten und zweiten Bindedomäne kodiert durch die Sequenzen wiedergegeben in SEQ ID NO: 5 und SEQ ID NO:6.

Ein Polypeptid, welches die Sequenzen wiedergegeben in SEQ ID NO: 5 und SEQ ID NO: 6 umfasst ist in SEQ ID NO: 7 oder SEQ ID NO:8 wiedergegeben.

Ein besonders bevorzugtes Polypeptid ist das PSMA-BiTE 1 Molekül, welches durch Aminosäuresequenz wiedergegeben in SEQ ID NO: 8 kodiert wird.

Offenbart ist weiterhin eine flüssige pharmazeutische Zusammensetzung umfassend ein Polypeptid, TRIS und Phosphat, wobei das Polypeptid die Aminosäuresequenz wiedergegeben in SEQ ID NO: 7 umfasst.

Eine besonders bevorzugte Ausführungsform der vorliegenden Erfindung ist eine flüssige pharmazeutische Zusammensetzung umfassend ein Polypeptid, TRIS und Phosphat, wobei das Polypeptid die Aminosäuresequenz wiedergegeben in SEQ ID NO: 8 umfasst.

Offenbart sind weiterhin Zusammensetzungen umfassend etwa 0,5 $\mu$g/ml, etwa 0,7 $\mu$g/ml, etwa 1 $\mu$g/ml, etwa 2 $\mu$g/ml, etwa 5 $\mu$g/ml, etwa 6 $\mu$g/ml, etwa 10 $\mu$g/ml, etwa 15 $\mu$g/ml, etwa 18 $\mu$g/ml, etwa 20 $\mu$g/ml, etwa 25 $\mu$g/ml, etwa 30 $\mu$g/ml, etwa 30 $\mu$g/ml, etwa 35 $\mu$g/ml, etwa 40 $\mu$g/ml, etwa 45 $\mu$g/ml, etwa 50 $\mu$g/ml, etwa 55 $\mu$g/ml, etwa 60 $\mu$g/ml, etwa 70 $\mu$g/ml, etwa 80 $\mu$g/ml, etwa 90 $\mu$g/ml, etwa 100 $\mu$g/ml, etwa 110 $\mu$g/ml, etwa 120 $\mu$g/ml, etwa 130 $\mu$g/ml, etwa 140 $\mu$g/ml, etwa 150 $\mu$g/ml, etwa 160 $\mu$g/ml, etwa 170 $\mu$g/ml, etwa 180 $\mu$g/ml, etwa 190 $\mu$g/ml, etwa 200 $\mu$g/ml, etwa 225 $\mu$g/ml, etwa 275 $\mu$g/ml, etwa 300 $\mu$g/ml, etwa 325 $\mu$g/ml, etwa 350 $\mu$g/ml, etwa 375 $\mu$g/ml, etwa 400 $\mu$g/ml, etwa 500 $\mu$g/ml, etwa 700 $\mu$g/ml, etwa 900 $\mu$g/ml, oder etwa 1000 $\mu$g/ml der oben genannten Polypeptide.

Offenbart sind weiterhin Zusammensetzungen umfassend 0,5 $\mu$g/ml, 0,7 $\mu$g/ml, 1 $\mu$g/ml, 2 $\mu$g/ml, 5 $\mu$g/ml, 6 $\mu$g/ml, 10 $\mu$g/ml, 15 $\mu$g/ml, 18 $\mu$g/ml, 20 $\mu$g/ml, 25 $\mu$g/ml, 30 $\mu$g/ml, 30 $\mu$g/ml, 35 $\mu$g/ml, 40 $\mu$g/ml, 45 $\mu$g/ml, 50 $\mu$g/ml, 55 $\mu$g/ml, 60 $\mu$g/ml, 70 $\mu$g/ml, 80 $\mu$g/ml, 90 $\mu$g/ml, 100 $\mu$g/ml, 110 $\mu$g/ml, 120 $\mu$g/ml, 130 $\mu$g/ml, 140 $\mu$g/ml, 150 $\mu$g/ml, 160 $\mu$g/ml, 170 $\mu$g/ml, 180 $\mu$g/ml, 190 $\mu$g/ml, 200 $\mu$g/ml, 225 $\mu$g/ml, 275 $\mu$g/ml, 300 $\mu$g/ml, 325 $\mu$g/ml, 350 $\mu$g/ml, 375 $\mu$g/ml, 400 $\mu$g/ml, 500 $\mu$g/ml, 700 $\mu$g/ml, 900 $\mu$g/ml, oder 1000 $\mu$g/ml der BiTE-Moleküle.

Offenbart sind weiterhin Zusammensetzungen umfassend etwa 1 mg/ml, etwa 1,3 mg/ml, etwa 1,5 mg/ml, etwa 1,8 mg/ml, etwa 2 mg/ml, etwa 2,3 mg/ml, etwa 2,5 mg/ml, etwa 2,8 mg/ml, oder etwa 3 mg/ml der BiTE-Moleküle.

Offenbart sind weiterhin Zusammensetzungen umfassend etwa 1 mg/ml, 1,3 mg/ml, 1,5 mg/ml, 1,8 mg/ml, 2 mg/ml, 2,3 mg/ml, 2,5 mg/ml, 2,8 mg/ml, oder 3 mg/ml der BiTE-Moleküle.

Offenbart ist weiterhin eine Zusammensetzung umfassend etwa 0,5 $\mu$g/ml bis etwa 1 $\mu$g/ml, von etwa 1 $\mu$g/ml bis etwa 5 $\mu$g/ml, von etwa 5 $\mu$g/ml bis etwa 10 $\mu$g/ml, von etwa 10 $\mu$g/ml bis etwa 20 $\mu$g/ml, von etwa 20 $\mu$g/ml bis etwa 50 $\mu$g/ml, von etwa 50 $\mu$g/ml bis etwa 90 $\mu$g/ml, von etwa 90 $\mu$g/ml bis etwa 120 $\mu$g/ml, von etwa 120 $\mu$g/ml bis etwa 150 $\mu$g/ml, von etwa 150 $\mu$g/ml bis etwa 180 $\mu$g/ml, von etwa 180 $\mu$g/ml bis etwa 200 $\mu$g/ml, von etwa 200 $\mu$g/ml bis etwa 250 $\mu$g/ml, von etwa 250 $\mu$g/ml bis etwa 280 $\mu$g/ml, von etwa 280 $\mu$g/ml bis etwa 300 $\mu$g/ml, oder von etwa 300 $\mu$g/ml bis etwa 350 $\mu$g/ml der BiTE-Moleküle.

Offenbart ist weiterhin eine Zusammensetzung umfassend 0,5 $\mu$g/ml bis 1 $\mu$g/ml, von 1 $\mu$g/ml bis 5 $\mu$g/ml, von 5 $\mu$g/ml bis 10 $\mu$g/ml, von 10 $\mu$g/ml bis 20 $\mu$g/ml, von 20 $\mu$g/ml bis 50 $\mu$g/ml, von 50 $\mu$g/ml bis 90 $\mu$g/ml, von 90 $\mu$g/ml bis 120 $\mu$g/ml, von 120 $\mu$g/ml bis 150 $\mu$g/ml, von 150 $\mu$g/ml bis 180 $\mu$g/ml, von 180 $\mu$g/ml bis 200 $\mu$g/ml, von 200 $\mu$g/ml bis 250 $\mu$g/ml, von 250 $\mu$g/ml bis 280 $\mu$g/ml, von 280 $\mu$g/ml bis 300 $\mu$g/ml, oder von 300 $\mu$g/ml bis 350 $\mu$g/ml der BiTE-Moleküle.

Offenbart ist weiterhin eine Zusammensetzung umfassend etwa 350 $\mu$g/ml bis etwa 1 mg/ml, von etwa 350 $\mu$g/ml bis etwa 1,3 mg/ml, von etwa 350 $\mu$g/ml bis etwa 1,5 mg/ml, von etwa 350 $\mu$g/ml bis etwa 1,8 mg/ml, von etwa 350 $\mu$g/ml bis etwa 2 mg/ml, von etwa 350 $\mu$g/ml bis etwa 2,3 mg/ml, von etwa 350 $\mu$g/ml bis etwa 2,5 mg/ml, von etwa 350 $\mu$g/ml bis etwa 2,8 mg/ml, oder von etwa 350 $\mu$g/ml bis etwa 3,0 mg/ml der BiTE-Moleküle.

Offenbart ist weiterhin eine Zusammensetzung umfassend 350 $\mu$g/ml bis 1 mg/ml, von 350 $\mu$g/ml bis 1,3 mg/ml, von 350 $\mu$g/ml bis 1,5 mg/ml, von 350 $\mu$g/ml bis 1,8 mg/ml, von 350 $\mu$g/ml bis 2 mg/ml, von 350 $\mu$g/ml bis 2,3 mg/ml, von 350 $\mu$g/ml bis 2,5 mg/ml, von 350 $\mu$g/ml bis 2,8 mg/ml, oder von 350 $\mu$g/ml bis 3,0 mg/ml der BiTE-Moleküle.

In einer weiteren Ausführungsform umfasst die erfindungsgemäße Zusammensetzung von 0,5 $\mu$g/ml bis 3,0 mg/ml der BiTE-Moleküle.

Offenbart ist weiterhin eine Zusammensetzung umfassend etwa 0,5 $\mu$g/ml bis etwa 3,0 mg/ml von etwa 0,5 $\mu$g/ml bis etwa 2,8 mg/ml, von etwa 0,5 $\mu$g/ml bis etwa 2,5 mg/ml, von etwa 0,5 $\mu$g/ml bis etwa 2,3 mg/ml, von etwa 0,5 $\mu$g/ml bis etwa 2,0 mg/ml, von etwa 0,5 $\mu$g/ml bis etwa 1,8 mg/ml, von etwa 0,5 $\mu$g/ml bis etwa 1,5 mg/ml, von etwa 0,5 $\mu$g/ml bis etwa 1,3 mg/ml, von etwa 0,5 $\mu$g/ml bis etwa 1,0 mg/ml, von etwa 0,5 $\mu$g/ml bis etwa 350 $\mu$g/ml, von etwa 0,5 $\mu$g/ml bis etwa 300 $\mu$g/ml, von etwa 0,5 $\mu$g/ml bis etwa 250 $\mu$g/ml, der BiTE-Moleküle.

In einer besonders bevorzugten Ausführungsform umfasst die erfindungsgemäße Zusammensetzung etwa 2 mg/ml der erfindungsgemäßen Polypeptide.

In einer weiteren Ausführungsform umfasst die erfindungsgemäße Zusammensetzung eine Kombination aus Tris(hydroxymethyl)-aminomethan (TRIS) und Phosphat als puffernde, den pH-Wert beeinflussende Agenzien.

In einer bevorzugten Ausführungsform umfasst die erfindungsgemäße Zusammensetzung TRIS in einer Konzentration von etwa 100 mM.

In einer bevorzugten Ausführungsform umfasst die erfindungsgemäße Zusammensetzung TRIS in einer Konzentration von 100 mM.

In einer bevorzugten Ausführungsform umfasst die erfindungsgemäße Zusammensetzung Phosphat in einer Konzentration von etwa 50 mM.

In einer bevorzugten Ausführungsform umfasst die erfindungsgemäße Zusammensetzung Phosphat in einer Konzentration von 50 mM.

In einer bevorzugten Ausführungsform umfasst die erfindungsgemäße Zusammensetzung etwa 2 mg/ml der erfindungsgemäßen Polypeptide sowie TRIS in einer Konzentration von 100 mM sowie Phosphat in einer Konzentration von 50 mM.

In einer bevorzugten Ausführungsform umfasst die erfindungsgemäße Zusammensetzung etwa 2 mg/ml der erfindungsgemäßen Polypeptide sowie Phosphat in einer Konzentration von etwa 50 mM,.

In einer bevorzugten Ausführungsform umfasst die erfindungsgemäße Zusammensetzung etwa 2 mg/ml der erfindungsgemäßen Polypeptide sowie Phosphat in einer Konzentration von 50 mM.

In einer bevorzugten Ausführungsform umfasst die erfindungsgemäße Zusammensetzung etwa 2 mg/ml der erfindungsgemäßen Polypeptide sowie TRIS in einer Konzentration von etwa 100 mM und Phosphat in einer Konzentration von etwa 50 mM.

In einer bevorzugten Ausführungsform umfasst die erfindungsgemäße Zusammensetzung etwa 2 mg/ml der erfindungsgemäßen Polypeptide sowie TRIS in einer Konzentration von etwa 100 mM und Phosphat in einer Konzentration von 50 mM.

In einer bevorzugten Ausführungsform umfasst die erfindungsgemäße Zusammensetzung etwa 2 mg/ml der erfindungsgemäßen Polypeptide sowie Phosphat in einer Konzentration von etwa 50 mM sowie TRIS in einer Konzentration von etwa 100 mM.

In einer bevorzugten Ausführungsform umfasst die erfindungsgemäße Zusammensetzung etwa 2 mg/ml der erfindungsgemäßen Polypeptide sowie Phosphat in einer Konzentration von etwa 50 mM sowie TRIS in einer Konzentration von 100 mM.

In einer bevorzugten Ausführungsform liegt der pH-Wert der erfindungsgemäßen Zusammensetzung in einem Bereich von etwa 5,0 bis etwa 7,0, oder in einem Bereich von etwa 5,0 bis etwa 6,5. Besonders bevorzugt liegt der pH-Wert der erfindungsgemäßen Zusammensetzung bei 6,0. Bevorzugt wird der pH-Wert der erfindungsgemäßen Zusammensetzung mit HCl eingestellt.

In einer weiteren Ausführungsform umfasst die erfindungsgemäße Zusammensetzung zusätzlich ein Netzmittel. Beispiele für Netzmittel sind nicht-ionosche Netzmittel wie Polysorbate (z.B. Polysorbat 20 oder 80); Polyoxamere (z.B. Polyoxamer 188); Triton; Natriumoctylglycosid; Lauryl-, Myristyl-, Lonoleyl-, oder Stearylsulfobetain; Lauryl-, Myristyl-, Lonoleyl-, oder Stearylsarcosin; Lineolyl-, Myrisyl-, oder Cetylbetain; Lauroamidopropyl-, Cocamidopropyl-, lineoamidopropyl, Myristamidopropyl-, Palmidopropyl-, oder Isostearamidopropylbetain; Polyethylglycol; Polypropylglycol; und Kopolymere aus Ethylen und Propylenglycol (z.B. Pluronics, PF68). In einer bevorzugten Ausführungsform ist das Netzmittel Polysorbat 80.

Offenbart ist weiterhin eine Zusammensetzung umfassend ein Netzmittel in einer Konzentration von 0.002% bis 0.1%, bevorzugt von 0.04% bis 0.1%.

Offenbart ist weiterhin eine Zusammensetzung umfassend Polysorbat 80 in einer Konzentration von 0.002% bis 0.1%, bevorzugt von 0.04% bis 0.1%. Besonders bevorzugt umfasst die erfindungsgemäße Zusammensetzung Polysorbat 80 in einer Konzentration von 0.04%.

In einer weiteren Ausführungsform umfasst die erfindungsgemäße Zusammensetzung zusätzlich ein Lyoprotektivum. In einer weiteren Ausführungsform umfasst die erfindungsgemäße Zusammensetzung zusätzlich einen Zucker oder einen Zuckeralkohol als Lyoprotektivum. Bei dem Lyoprotektivum handelt es sich bevorzugt um Trehalose. In einer bevorzugten Ausführungsform umfasst die erfindungsgemäße Zusammensetzung das Lyoprotektivum in einer Konzentration von 2% bis 10%, besonders bevorzugt von 4%.

In einer bevorzugten Ausführungsform umfasst die erfindungsgemäße Zusammensetzung Trehalose in einer Konzentration von 2% bis 10%, besonders bevorzugt von 4%.

Offenbart sind Zusammensetzungen umfassend etwa 0,5 $\mu$g/ml bis etwa 2 mg/ml der PSMA-BiTE1-Moleküle sowie TRIS in einer Konzentration von etwa 50 mM bis etwa 200 mM, Phosphat in einer Konzentration von etwa 20 mM bis etwa 100 mM, Polysorbat 80 in einer Konzentration von 0.04% und Trehalose in einer Konzentration von 4%, wobei der pH-Wert bei 6,0 liegt.

Offenbart sind weiterhin Zusammensetzungen umfassend etwa 0,5 $\mu$g/ml bis etwa 2 mg/ml der PSMA-BiTEl- Moleküle sowie TRIS in einer Konzentration von etwa 50 mM bis etwa 200 mM, Phosphat in einer Konzentration von etwa 50 mM, Polysorbat 80 in einer Konzentration von 0.04% und Trehalose in einer Konzentration von 4%, wobei der pH-Wert bei 6,0 liegt.

Offenbart sind weiterhin Zusammensetzungen umfassend etwa 0,5 $\mu$g/ml bis etwa 2 mg/ml der PSMA-BiTEl- Moleküle sowie TRIS in einer Konzentration von etwa 100 mM, Phosphat in einer Konzentration von etwa 20 mM bis etwa 100 mM, Polysorbat 80 in einer Konzentration von 0.04% und Trehalose in einer Konzentration von 4%, wobei der pH-Wert bei 6,0 liegt.

Offenbart ist weiterhin eine erfindungsgemäße Zusammensetzung umfassend etwa 50$\mu$g/ml bis etwa 2 mg/ml der PSMA-BiTEl-Moleküle sowie TRIS in einer Konzentration von etwa 100 mM, Phosphat in einer Konzentration von etwa 50 mM, Polysorbat 80 in einer Konzentration von 0.04% und Trehalose in einer Konzentration von 4%, wobei der pH-Wert bei 6,0 liegt.

Offenbart ist weiterhin eine Zusammensetzung umfassend etwa 50$\mu$g/ml bis etwa 1 mg/ml der PSMA-BiTEl- Moleküle sowie TRIS in einer Konzentration von etwa 100 mM, Phosphat in einer Konzentration von etwa 50 mM, Polysorbat 80 in einer Konzentration von 0.04% und Trehalose in einer Konzentration von 4%, wobei der pH-Wert bei 6,0 liegt.

Offenbart ist weiterhin eine Zusammensetzung umfassend etwa 100$\mu$g/ml bis etwa 500 $\mu$g/ml der PSMA-BiTEl- Moleküle sowie TRIS in einer Konzentration von etwa 100 mM, Phosphat in einer Konzentration von etwa 50 mM, Polysorbat 80 in einer Konzentration von 0.04% und Trehalose in einer Konzentration von 4%, wobei der pH-Wert bei 6,0 liegt.

Bevorzugt umfasst die erfindungsgemäße Zusammensetzung etwa 2 mg/ml der PSMA-BiTE1-Moleküle sowie TRIS in einer Konzentration von etwa 100 mM, Phosphat in einer Konzentration von etwa 50 mM, Polysorbat 80 in einer Konzentration von 0.04% und Trehalose in einer Konzentration von 4%, wobei der pH-Wert bei 6,0 liegt.

Bevorzugt umfasst die erfindungsgemäße Zusammensetzung etwa 2 mg/ml der PSMA-BiTE1-Moleküle sowie TRIS in einer Konzentration von etwa 100 mM, $Na_2HPO_4$ in einer Konzentration von etwa 50 mM, Polysorbat 80 in einer Konzentration von 0.04% und Trehalose in einer Konzentration von 4%, wobei der pH-Wert bei 6,0 liegt.

Konzentrationsangaben in Prozent (%) beziehen sich auf die Massenkonzentration (Masse / Volumen).

Zusätzlich können die erfindungsgemäßen Zusammensetzungen noch weitere pharmazeutisch akzeptierte Zusätze enthalten (Remington's Pharmaceutical Sciences; 18. Ausgabe, Mack Publishing Co., Easton, PA, USA). Solche Zusätze sind z.B. Konservierungsstoffe oder Antioxidantien. Als Antioxidantien können z.B. Ascorbat, Methionin, Vitamin E, oder Natriummetabisulfit verwendet werden. Konservierungsstoffe sind z.B. Substanzen, die das Wachstum von Mikroorganismen unterdrücken oder verlangsamen. Eine solche Substanz ist z.B. Thimerosal.

Eine Ausführungsform der vorliegenden Erfindung ist ein Feststoffgemisch, das durch Lyophilisation der erfindungsgemäßen Zusammensetzung hergestellt, oder zumindest durch Lyophilisation dieser Zusammensetzung herstellbar ist.

Eine bevorzugte Ausführungsform der vorliegenden Erfindung ist ein Lyophilisat, herstellbar durch Gefriertrocknung einer erfindungsgemäßen Zusammensetzung.

Eine bevorzugte Ausführungsform der vorliegenden Erfindung ist ein Lyophilisat, hergestellt durch Gefriertrocknung einer erfindungsgemäßen Zusammensetzung nach dem im Beispiel 16 beschriebenen Protokoll.

In einer weiteren Ausführungsform wird die erfindungsgemäße Zusammensetzung bereitgestellt, indem das lyophilisierte Feststoffgemisch durch Lösung in einem geeigneten flüssigen Medium rekonstituiert wird.

In einer bevorzugten Ausführungsform wird die erfindungsgemäße Zusammensetzung bereitgestellt, indem das lyophilisierte Feststoffgemisch durch Lösung in Wasser, bevorzugt sterilem Wasser, rekonstituiert wird.

Ein weiterer Gegenstand der Erfindung ist ein Erzeugnis, welches eine der erfindungsgemäßen Zusammensetzungen und bevorzugt auch eine Anleitung zum Gebrauch enthält. In einer Ausführungsform umfasst das Erzeugnis einen Behälter, der eine der oben aufgeführten Zusammensetzungen enthält. Verwendbare Behälter sind z.B. Flaschen, Ampullen, Röhrchen oder Spritzen. Die Behälter können z.B. aus Glas oder Kunststoff sein. Spritzen können eine Injektionsnadel, z.B. aus Metall, umfassen.

In einer Ausführungsform ist der Behälter eine Spritze. In einer weiteren Ausführungsform ist die Spritze in einer Injektionsvorrichtung enthalten. In einer bevorzugten Ausführungsform ist die Injektionsvorrichtung ein Autoinjektor. Ein Autoinjektor kann als ein Injektionsgerät beschrieben werden, welches nach Aktivierung seinen Inhalt ohne zusätzliche Handlung des Patienten oder einer anderen Person verabreicht. In der vorliegenden Erfindung ist die bevorzugte Verabreichung subkutan.

Die erfindungsgemäßen Zusammensetzungen weisen im Vergleich zu den im Stand der Technik verfügbaren Formulierungen für BiTE Moleküle eine erhöhte Stabilität und signifikant erhöhte Bioverfügbarkeit auf. Die erfindungsgemäßen Zusammensetzungen sind aufgrund dieses Eigenschaftsprofils besonders zur parenteralen Applikation geeignet. Parenterale Applikationen sind unter anderem die intravenöse Injektion oder Infusion, intraarterielle Injektion oder Infusion (in eine Arterie), intramuskuläre Injektion, intrathekale Injektion, subkutane Injektion, intraperitoneale Injektion oder Infusion, intraossäre Applikation oder Injektion in ein Gewebe. Insbesondere sind die erfindungsgemäßen Zusammensetzungen zur subkutanen Applikation geeignet. Eine Ausführungsform der erfindungsgemäßen Zusammensetzung ist dadurch gekennzeichnet, dass die Bioverfügbarkeit des Polypeptids nach subkutaner Gabe der Zusammensetzung >60% beträgt.

Die erfindungsgemäßen Zusammensetzungen besitzen wertvolle pharmakologische Eigenschaften und können zur Vorbeugung und Behandlung von Erkrankungen bei Menschen und Tieren verwendet werden.

Die erfindungsgemäßen Zusammensetzungen sind zur Behandlung von hyperproliferativen Erkrankungen beim Menschen und bei Säugetieren allgemein geeignet. Zu den hyperproliferativen Erkrankungen, zu deren Behandlung die erfindungsgemäßen Zusammensetzungen eingesetzt werden können, zählt insbesondere die Gruppe der Krebs- und Tumorerkrankungen. Hierunter werden im Rahmen der vorliegenden Erfindung insbesondere die folgenden Erkrankungen verstanden, ohne jedoch auf sie beschränkt zu sein: Brustkarzinome und Brusttumore (ductale und lobuläre Formen, auch *in situ),* Atemwegstumore (kleinzelliges und nicht-kleinzelliges Karzinom, Bronchialkarzinom), Hirntumore (z.B. des Hirnstamms und des Hypothalamus, Astrocytoma, Medulloblastoma, Ependymoma sowie neuro-ectodermale und pineale Tumore), Tumore der Verdauungsorgane (Speiseröhre, Magen, Gallenblase, Dünndarm, Dickdarm, Rektum), Lebertumore (u.a. hepatozelluläres Karzinom, Cholangiokarzinom und gemischt-hepatozelluläres Cholangiokarzinom), Tumore des Kopf- und Halsbereiches (Larynx, Hypopharynx, Nasopharynx, Oropharynx, Lippen und Mundhöhle), Hauttumore (Plattenepithelkarzinom, Kaposi-Sarkom, malignes Melanom, Merkelzell-Hautkrebs und nicht-melanomartiger Hautkrebs), Tumore der Weichteile (u.a. Weichteilsarkome, Osteosarkome, maligne fibröse Histiozytome, Lymphosarkome und Rhabdomyosarkome), Tumore der Augen (u.a. intraokuläres Melanom und Retinoblastom), Tumore der endokrinen und exokrinen Drüsen (z.B. thyroide und parathyroide Drüsen, Bauchspeicheldrüse und Speicheldrüse), Tumore des Harntrakts (Blasen-, Penis-, Nieren-, Nierenbecken- und Harnleitertumore) sowie Tumore der reproduktiven Organe (Endometrium-, Zervix-, Ovarial-, Vaginal-, Vulva- und Uteruskarzinome der Frau sowie Prostata- und Hodenkarzinome des Mannes). Dazu gehören auch proliferative Bluterkrankungen in solider Form und als zirkulierende Blutzellen, wie Lymphome, Leukämien und myeloproliferative Erkrankungen, z.B. akute myeloide, akute lymphoblastische, chronisch-lymphozytische, chronisch-myelogene und Haarzell-Leukämie, sowie AIDS-korrelierte Lymphome, Hodgkin-Lymphome, Non-Hodgkin-Lymphome, kutane T-Zell-Lymphome, Burkitt-Lymphome und Lymphome im zentralen Nervensystem.

Bevorzugte Erkrankungen, zu deren Behandlung die erfindungsgemäßen Zusammensetzungen eingesetzt werden können, sind Karzinome und/oder Metastasen die das PSMA Antigen exprimieren.

Eine besonders bevorzugte Erkrankung, zu deren Behandlung die erfindungsgemäßen Zusammensetzungen eingesetzt werden können, ist ausgewählt aus der Gruppe bestehend aus Prostatakarzinom, Knochenmetastasen des Prostatakarzinoms und Weichteilmetastasen des Prostatakarzinoms.

Eine weiter besonders bevorzugte Erkrankung, zu deren Behandlung die erfindungsgemäßen Zusammensetzungen eingesetzt werden können, ist das Prostatakarzinom.

Diese gut beschriebenen Krankheiten des Menschen können mit vergleichbarer Ätiologie auch in anderen Säugetieren vorkommen und dort mit den Zusammensetzungen der vorliegenden Erfindung behandelt werden.

Der Begriff "Behandlung" oder "behandeln" wird im Rahmen dieser Erfindung konventionell verwendet und bedeutet die Versorgung, Pflege und Betreuung eines Patienten mit dem Ziel, eine Krankheit oder gesundheitliche Abweichung zu bekämpfen, zu verringern, abzuschwächen oder zu erleichtern und die Lebensbedingungen zu verbessern, die durch diese Krankheit beeinträchtigt werden, wie beispielsweise bei einer Krebserkrankung.

Weiterer Gegenstand der vorliegenden Erfindung ist somit die Verwendung der erfindungsgemäßen Zusammensetzungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Zusammensetzungen zur Herstellung eines Arzneimittels zur Behandlung und/oder Prävention von Erkrankungen, insbesondere der zuvor genannten Erkrankungen.

Weiterer Gegenstand der vorliegenden Erfindung ist die Verwendung der erfindungsgemäßen Zusammensetzungen in einem Verfahren zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge einer der erfindungsgemäßen Zusammensetzungen.

In einer bevorzugten Ausführungsform ist die Behandlung und/oder Prävention eine parenterale Applikation der erfindungsgemäßen Zusammensetzung. Besonders bevorzugt ist die subkutane Applikation.

Die erfindungsgemäßen Zusammensetzungen können allein oder bei Bedarf in Kombination mit einer oder mehreren anderen pharmakologisch wirksamen Substanzen eingesetzt werden, solange diese Kombination nicht zu unerwünschten und inakzeptablen Nebenwirkungen führt. Weiterer Gegenstand der vorliegenden Erfindung sind daher Arzneimittel, enthaltend mindestens eine der erfindungsgemäßen Zusammensetzungen und einen oder mehrere weitere Wirkstoffe, insbesondere zur Behandlung und/oder Prävention der zuvor genannten Erkrankungen. Beispielsweise können die Verbindungen der vorliegenden Erfindung mit bekannten anti-hyperproliferativen, zytostatischen oder zytotoxischen Substanzen zur Behandlung von Krebserkrankungen kombiniert werden.

Ein weiterer Gegenstand der Erfindung ist die Verwendung der vorgenannten Zusammensetzungen in einem therapeutischen Verfahren, wobei die Zusammensetzung geeignet ist für parenterale Applikationsformen, wie die intravenöse Injektion oder Infusion, intraarterielle Injektion oder Infusion (in eine Arterie), intramuskuläre Injektion, intrathekale Injektion, subkutane Injektion, intraperitoneale Injektion oder Infusion, intraossäre Applikation oder Injektion in ein Gewebe.

Ein weiterer Gegenstand der Erfindung ist die Verwendung der vorgenannten Zusammensetzungen in einem Verfahren zur therapeutischen Behandlung von zellproliferativen Erkrankungen der Prostata.

Ein weiterer Gegenstand der Erfindung ist die Verwendung der vorgenannten Zusammensetzungen in einem Verfahren zur therapeutischen Behandlung von zellproliferativen Erkrankungen der Prostata, wobei die Zusammensetzung geeignet ist für die subkutane Applikation.

Ein weiterer Gegenstand der Erfindung ist die Verwendung der vorgenannten Zusammensetzungen in einem Verfahren zur therapeutischen Behandlung von zellproliferativen Erkrankungen der Prostata, wobei die Zusammensetzung durch subkutane Applikation verabreicht wird.

Ein weiterer Gegenstand der Erfindung ist eine Methode zur Stabilisierung von Polypeptiden, umfassend die Herstellung einer der vorgenannten Zusammensetzungen, die neben den Polypeptiden, mindestens TRIS und Phosphat enthält und einen pH-Wert von 6.0 besitzt.

Ein weiterer Gegenstand der Erfindung ist ein Kit, der die vorgenannten Zusammensetzungen umfasst.

Bevorzugte Verbindungen im Sinne der vorliegenden Erfindung sind pharmazeutische Verbindungen.

Ausführungsformen

Eine Ausführungsform der vorliegenden Erfindung umfasst eine flüssige pharmazeutische Zusammensetzung umfassend ein Polypeptid, TRIS und Phosphat wobei das Polypeptid zwei scFv Antikörperbindedomänen umfasst, wobei die erste scFv Bindedomäne an das humane CD3 epsilon binden kann.

In einer weiteren Ausführungsform der Zusammensetzung kann die zweite Bindedomäne des Polypeptids an ein Zelloberflächenantigen binden.

In einer weiteren Ausführungsform der Zusammensetzung umfasst das Polypeptid eine zweite Bindedomäne, die an ein Oberflächenantigen einer Krebszelle binden kann.

In einer weiteren Ausführungsform ist das Oberflächenantigen, an das die zweite Bindedomäne des Polypeptids binden kann das Prostata-Spezifische Membran-Antigen (PSMA).

In einer weiteren Ausführungsform der Zusammensetzung hat das Polypeptid die Anordnung (VH-VL)Bindedomäne2-(VH - VL)Bindedomänel.

In einer weiteren Ausführungsform der Zusammensetzung umfasst die erste Bindedomäne des Polypeptids die Aminosäuresequenz wiedergegeben in SEQ ID NO: 5.

In einer weiteren Ausführungsform der Zusammensetzung umfasst die zweite, PSMA-bindende Bindedomäne des Polypeptids die Aminosäuresequenz wiedergegeben in SEQ ID NO: 6.

In einer weiteren Ausführungsform umfasst die Zusammensetzung ein Polypeptid, TRIS und Phosphat, wobei das Polypeptid die Aminosäuresequenz wiedergegeben in SEQ ID NO: 8 umfasst.

In einer weiteren Ausführungsform enthält die Zusammensetzung das Polypeptid in einer Konzentration von 0,5 $\mu$g/mL bis 3.0 mg/ml.

In einer weiteren Ausführungsform enthält die Zusammensetzung das Polypeptid in einer Konzentration von etwa 2 mg/ml.

In einer weiteren Ausführungsform enthält die Zusammensetzung 100 mM TRIS und 50 mM Phosphat.

In einer weiteren Ausführungsform liegt der pH-Wert der Zusammensetzung in einem Bereich von etwa 5,0 bis etwa 7,0.

In einer weiteren Ausführungsform liegt der pH-Wert der Zusammensetzung bei etwa 6,0.

In einer weiteren Ausführungsform wird der pH-Wert der Zusammensetzung mit Salzsäure eingestellt.

In einer weiteren Ausführungsform enthält die Zusammensetzung zusätzlich ein Netzmittel.

In einer weiteren Ausführungsform ist das Netzmittel Polysorbat 80.

In einer weiteren Ausführungsform enthält die Zusammensetzung zusätzlich 0,04% Polysorbat 80.

In einer weiteren Ausführungsform enthält die Zusammensetzung zusätzlich ein Lyoprotektivum.

In einer weiteren Ausführungsform enthält die Zusammensetzung als Lyoprotektivum Trehalose.

In einer weiteren Ausführungsform enthält die Zusammensetzung zusätzlich 4% bis 10%Trehalose.

In einer weiteren Ausführungsform enthält die Zusammensetzung zusätzlich etwa 4% Trehalose.

Eine Ausführungsform der vorliegenden Erfindung umfasst ein Feststoffgemisch, herstellbar durch Lyophilisation der flüssigen Zusammensetzung.

In einer weiteren Ausführungsform wird die Zusammensetzung durch Lösung eines lyophilisierten Feststoffgemisches nach Anspruch 43 in einem geeigneten flüssigen Medium rekonstituiert.

In einer weiteren Ausführungsform beträgt die Bioverfügbarkeit des Polypeptids nach subkutaner Gabe der Zusammensetzung >60%.

In einer weiteren Ausführungsform findet die Zusammensetzung Anwendung in einem therapeutischen Verfahren.

In einer weiteren Ausführungsform findet die Zusammensetzung Anwendung in einem therapeutischen Verfahren, wobei dieses Verfahren die parenterale Applikation der Zusammensetzung umfasst.

In einer weiteren Ausführungsform findet die Zusammensetzung Anwendung in einem Verfahren zur therapeutischen Behandlung hyperproliferativer Erkrankungen.

In einer weiteren Ausführungsform findet die Zusammensetzung Anwendung in einem Verfahren zur therapeutischen Behandlung hyperproliferativer Erkrankungen der Prostata.

In einer weiteren Ausführungsform findet die Zusammensetzung Anwendung in einem Verfahren zur therapeutischen Behandlung hyperproliferativer Erkrankungen der Prostata, wobei das Verfahren die subkutane Applikation der Zusammensetzung umfasst.

In einer weiteren Ausführungsform umfasst die vorliegende Erfindung eine Methode zur Stabilisierung von Polypeptiden, umfassend die Herstellung einer Zusammensetzung, die neben den Polypeptiden mindestens TRIS und Phosphat enthält und einen pH-Wert von 6.0 besitzt.

In einer weiteren Ausführungsform umfasst die vorliegende Erfindung einen Kit, umfassend die vorbeschriebene Zusammensetzung.

**Beispiele**

**Beispiel 1: Pufferscreening zur Verbesserung der Thermostabilität der PSMA-BiTE1-Moleküle**

Mithilfe der Differential-Scanning-Fluorimetrie (DSF) wurde der mittlere Schmelzpunkt ($T_{m1}$) der PSMA-BiTEl-Proteindomäne mit dem niedrigsten Molekulargewicht in unterschiedlichen Puffersystemen gemessen. Dieser ist ein Maß für die Stabilität des untersuchten Proteins in den verschiedenen Puffersystemen: Je höher der $T_m$-Wert, desto höher ist auch die Thermostabilität des Proteins. Je thermostabiler ein Protein ist, desto besser eignet es sich zur Herstellung stabiler pharmazeutischer Formulierungen.

Für das Pufferscreening wurden Standardpuffer in einem pH-Bereich von pH 5,0 bis 8,0 eingesetzt. Die PSMA-BiTEl-Konzentration der hergestellten Formulierungen betrug etwa 0,2 mg/ ml. Der mittlere Schmelzpunkt wurde mit der DSF-Methode ermittelt.

*Tab. 1: PSMA-BITE1 (0,2 mg/ml) in verschiedenen Puffersystemen (50 mM)*

| pH | $Na_2HPO_4$ Tm1 [°C] | Citrat Tm1 [°C] | Histidin Tm1 [°C] | Glycin Tm1 [°C] | Lysin Tm1 [°C] |
|---|---|---|---|---|---|
| 5,0 | 61,2 | 63,6 | - | - | - |
| 5,5 | 63,5 | 63,1 | 60,8 | - | - |
| 6,0 | 63,4 | 63,5 | 61,4 | 60,2 | 61,6 |
| 6,5 | 63,8 | 64,6 | 60,7 | 58,7 | 61,5 |
| 7,0 | 63,3 | - | 60,4 | 60,7 | 61,4 |
| 7,5 | 63,1 | - | 57,7 | 61,4 | 61,4 |
| 8,0 | 63,0 | - | 59,3 | - | - |

Die auf Citrat und $Na_2HPO_4$ basierenden Puffersysteme zeigten einen positiven Effekt im Hinblick auf die Erhöhung des Schmelzpunktes der PSMA-BiTEl-Proteindomämene. Die positiven Effekte von Citrat und $Na_2HPO_4$ wurden in weiteren Versuchen weiterverfolgt. Tris(hydroxymethyl)-aminomethan (TRIS)-basierte Puffer führten alleine nicht zu einer deutlichen Erhöhung des Proteinschmelzpunktes (Tab. 2).

*Tab. 2: PSMA-BiTE1 (0,2 mg/ ml) in verschiedenen Puffersystemen (50 mM) (Fortsetzung von Tab. 1)*

| pH | HEPES Tm1 [°C] | TRIS Tm1 [°C] | MOPS Tml [°C] | Acetat Tm1 [°C] |
|---|---|---|---|---|
| 6,5 | - | 61,7 | 62,5 | 61,9 |
| 7,0 | 62,2 | 61,4 | 61,8 | - |
| 7,5 | 61,4 | - | 62,6 | - |
| 8,0 | 61,4 | - | 62,4 | - |

Wie Tab. 1, zeigt auch Tab. 2 den mittels Differential-Scanning-Fluorimetrie ermittelten mittleren Schmelzpunkt (Tm) der PSMA-BiTEl-Proteindomäne mit dem niedrigsten Molekulargewicht in unterschiedlichen Puffersystemen. Die Tm-Werte lagen zwischen 57,7°C und 64,6°C. Über die Kombination verschiedener Puffersysteme waren keine höheren Tm Werte zu erreichen.

Das PSMA-BiTEl-Formulierungen in Phosphatpuffer bei pH 5,5-6,5 und in Citratpuffer bei pH 5,0-6,5 zeigten die höchsten Schmelzpunkte (Tm > 63,0°C nach DSF-Methode).

**Beispiel 2: Der Einfluss nichtionischer Tenside auf die PSMA-BiTE1-Aggregatbildung**

Die PSMA-BiTEl-Moleküle bildeten nach dem Schüttelstresstest in allen getesteten Puffersystemen Aggregate. Die Effizienz, mit der aggregierte PSMA-BiTEl-Moleküle eine T-Tellaktivierung bewirken, ist nicht oder nur eingeschränkt vorhersagbar, bzw. kontrollierbar. Daher war es zwingend notwendig einen Stabilisator zu finden, der die Aggregatbildung durch Schüttelstress oder die Einwirkung von Scherkräften verhindert. Im Schüttelstresstest zeigte sich, dass verschiedene nichtionische Tenside (z.B. Polysorbat 80 oder 20) die PSMA-BiTEl-Moleküle stabilisieren und eine Aggregation verhindern können. Tensidkonzentrationen zwischen 0,01 bis 0,04% (m/V) waren für die Stabilisierung ausreichend (siehe Tabelle 3-7).

**Beispiel 3: PSMA-BiTE1-Dimer-Bildung in Anwesenheit mehrwertiger Kationen**

Ein Anstieg des Anteils an PSMA-BiTEl-Aggregaten während der Aufkonzentrierung von PSMA-BiTE1-Formulierungen konnte durch Zusatz von nichtionischen Tensiden nicht verhindert werden.

Deshalb wurde die elektrostatische Stabilisierung der PSMA-BiTEl-Moleküle in Anwesenheit von mehrwertigen Kationen (z.B. $Mg^{2+}$ und $Ca^{2+}$) untersucht. Mehrwertige Ionen haben einen direkten Einfluss auf das Oberflächenpotential gelöster Proteine und können so stabilisierend oder auch destabilisierend wirken.

Die PSMA-BiTEl-Moleküle ließen sich mit Hilfe von Magnesiumchlorid stabilisieren. Der Anteil an PSMA-BiTEl-Dimeren stieg nach der Aufkonzentrierung nur geringfügig und blieb unter <3% (Tabelle 3). Die Messung der Monomer- und Dimeranteile erfolgte über Größenausschluss-Chromatographie (Size Exclusion Chromatography, SEC).

*Tab. 3: PSMA-BiTE1-Moleküle nach Aufkonzentrierung (in 50 mM $Na_2HPO_4$ und 50 mM Lysin, pH 7,3)*

| Zusätze während Aufkonzentrierung | Proteingehalt [mg/ ml] | Monomer [%] | Dimer [%] |
|---|---|---|---|
| - | 2,04 | 95,7 | 4,3 |
| 0,04% (m/V) Polysorbat 20 | 2,09 | 91,2 | 8,8 |
| 100mM $MgCl_2$ | 2,13 | 98,1 | 1,9 |
| SEC = Größenausschluss-Chromatographie | | | |

Allerdings ist die Zugabe von anorganischen Salzen in höheren Konzentrationen pharmazeutisch bedenklich, bzw. stellen diese Zusätze eine Herausforderung bei der Gefriertrocknung dar. Aus diesem Grund wurden alternative Hilfsstoffe gesucht, welche die PSMA-BiTEl-Moleküle stabilisieren und gleichzeitig pharmazeutisch unbedenklich sind.

**Beispiel 4**: **Identifizierung alternativer Hilfsstoffe zur Stabilisierung der PSMA-BiTE1-Monomere**

Eher zufällig wurden u. a. verschiedene Aminosäuren und deren Derivate in die Tests zur Stabilisierung der PSMA-BiTEl-Monomere in höheren Konzentrationen mit eingeschlossen. Aminosäuren und ihre Derivate sind keine anorganischen Salze und werden daher als pharmazeutisch unbedenklich eingestuft. Einige dieser Substanzen (z.B. Lysin) zeigten überraschenderweise einen positiven Einfluss auf die Stabilität der PSMA-BiTEl-Moleküle während und nach der Aufkonzentrierung (Tabelle 4).

*Tab. 4:* PSMA-BiTE1-*Moleküle nach Aufkonzentrierung bei pH 7,3*

| Puffer | Protein-gehalt [mg/ml] | SEC Monomer [%] | SEC Dimer [%] | DLS Median [nm] | Zustand nach Schüttel -stress |
|---|---|---|---|---|---|
| 50mM $Na_2HPO_4$ + 50mM Lysin 0,02% (m/V) Polysorbat 20, 10% Trehalose | 1,94 | 95,1 | 4,9 | 11 | trübe |
| 50mM $Na_2HPO_4$ + 100mM Lysin 0,04% (m/V) Polysorbat 20, 10% Trehalose | 1,88 | 97,5 | 2,5 | 9 | trübe |
| 10mM $Na_2HPO_4$ + 50mM Lysin + 100mM Histidin 0,04% (m/V) Polysorbat 20, 10% Trehalose | 2,15 | 92,4 | 7,6 | 10 | Ok |
| SEC = Größenausschluss-Chromatographie; DLS = Dynamische Lichtstreuung; eine trübe Lösung nach Schüttelstress zeigt einen hohen Anteil an BiTE-Aggregaten an, eine klare oder geringfügig getrübte Lösung (Zustand "ok") zeigt einen niedrigen Grad der Aggregation an. | | | | | |

Durch Zusatz von Lysin und Histidin in einem Phosphatpuffer ließen sich die PSMA-BiTE1-Moleküle aufkonzentrieren, ohne die Ausbildung inakzeptabler Dimer-Anteile (≥5%). Die Formulierungen, bei denen der Dimeranteil niedrig (<5%) war, destabilisierten jedoch während des Schüttelstresstests, was an der Eintrübung der Lösung erkennbar war (Ansätze 1 und 2; "trübe" zeigt Aggregation an, "ok" zeigt keine oder geringe Aggregation an).

**Beispiel 5: Einfluss des pH-Wertes auf die Stabilität der PSMA-BiTEl-Moleküle**

Um den Einfluss des pH-Wertes auf die Dimerbildung zu untersuchen, wurde eine Formulierung mit pH 6,0 hergestellt. Diese zeigte einen Dimeranteil, der vergleichbar mit denen der Formulierungen bei pH 7,3 war. Darüber hinaus war der Dimeranteil auch im Schüttelstress stabil, was sich an der fehlenden Eintrübung ablesen ließ (Tabelle 5). Der positive Einfluss von pH 6,0 wurde für die weitere Suche von geeigneten Stabilisatoren und Formulierungen verwendet.

*Tab. 5: PSMA-BiTE1-Moleküle nach Aufkonzentrierung bei pH 6,0*

| Puffer bei pH 6,0 | Protein-gehalt [mg/ml] | SEC Monomer [%] | SEC Dimer [%] | DLS Median [nm] | Zustand nach Schüttelstress |
|---|---|---|---|---|---|
| 50mM $Na_2HPO_4$ + 50mM Lysin 0,04% (m/V) Polysorbat 20, 10% (m/V) Trehalose | 1,62 | 95,7 | 4,3 | 8 | ok |
| SEC = Größenausschluss-Chromatographie; DLS = Dynamische Lichtstreuung | | | | | |

Erstaunlich war die PSMA-BiTEl-Stabilität im Phosphatpuffer bei pH 6,0. Die Moleküle ließen sich in einer Formulierung umfassend 50mM $Na_2HPO_4$, 50mM Lysin 0,04% Polysorbat 20 und 10% Trehalose bei pH 6,0 plötzlich auf 1,6 mg/ ml aufkonzentrieren, ohne durch die Einwirkung von Schüttelstress zu aggregieren.

**Beispiel 6: Untersuchung von Pufferkombinationen**

In einem weiteren Versuch wurde ein möglicher synergistischer Effekt bzgl. einer Erhöhung der Stabilität der PSMA-BiTEl-Moleküle durch Zusätze wie Arginin, TEA oder TRIS in Kombination mit Phosphat untersucht. Der niedrigste Anteil an PSMA-BiTEl-Dimeren von 0,8% trat bei der Pufferkombination 50 mM $Na_2HPO_4$, 100 mM TRIS bei pH 6,0 auf. Die Formulierung war auch nach Schüttelstress ausreichend stabil, was an der ausbleibenden Eintrübung der Lösung zu erkennen war (Tabelle 6). Überraschend war die stabilisierende Wirkung von TRIS, da der Zusatz von Arginin oder TEA, die beide bekannt sind für ihre stabilisierende (d.h. Aggregationsreduzierende) Wirkung bei Proteinen, bei den PSMA-BiTE-Molekülen keine stabilisierende Wirkung hatte.

*Tab. 6: PSMA-BiTE1-Moleküle nach Aufkonzentrierung mit verschiedenen Zusätzen bei pH 6,0*

| Puffer bei pH 6,0 | Protein-gehalt [mg/ml] | SEC Monomer [%] | SEC Dimer [%] | DLS Median [nm] | Zustand nach Schüttelstress |
|---|---|---|---|---|---|
| 50mM $Na_2HPO_4$ 0,04% (m/V) Polysorbat 80, 4% (m/V) Trehalose | 1,82 | 96,4 | 3,3 | 13 | ok |
| 50mM $Na_2HPO_4$ + 100mM TRIS 0,04% (m/V) Polysorbat 80, 4% (m/V) Trehalose | 1,81 | 99,2 | 0,8 | 12 | ok |
| 50mM $Na_2HPO_4$ + 100mM Arginin 0,04% (m/V) Polysorbat 80, 4% (m/V) Trehalose | 2,20 | 96,2 | 3,5 | 15 | ok |
| 50mM $Na_2HPO_4$ + 100mM TEA 0,04% (m/V) Polysorbat 80, 4% (m/V) Trehalose | 1,88 | 96,2 | 3,4 | 11 | ok |
| SEC = Größenausschluss-Chromatographie; DLS = Dynamische Lichtstreuung | | | | | |

Wie in Beispiel 1 gezeigt, führte der Einsatz von Citratpuffer zu einer Erhöhung der Thermostabilität von PSMA-BiTEl-Molekülen. Nach Aufkonzentrierung der Ansätze lag der Dimeranteil jedoch in den Citrat-gepufferten Ansätzen wesentlich höher als in denjenigen mit Phosphatpuffer (Tabelle 7 im Vergleich zu Tabelle 6). Phosphatpuffer ist folglich besser geeignet als Citratpuffer, um die PSMA-BiTEl-Dimerbildung während der Aufkonzentrierung zu minimieren. Außerdem kann Citrat in einer Formulierung zu Glas-Delamination führen und sollte nicht mehr eingesetzt werden.

*Tab. 7:* PSMA-BiTE1-Molekülenach *Aufkonzentrierung bei pH 6,0*

| Puffer | Protein-gehalt [mg/ml] | SEC Monomer [%] | SEC Dimer [%] | DLS Median [nm] | Zustand nach Schüttel -stress |
|---|---|---|---|---|---|
| *50mM Citrat 0,04% (m/V) Polysorbat 80, 4% (m/V) Trehalose* | 1,90 | 93,4 | 6,4 | 11 | ok |
| *50mM Citrat + 100mM TRIS 0,04% (m/V) Polysorbat 80, 4% (m/V) Trehalose* | 1,95 | 92,9 | 7,0 | 12 | ok |
| SEC = Größenausschluss-Chromatographie; DLS = Dynamische Lichtstreuung | | | | | |

### Beispiel 7: Thermostabilität der PSMA-BiTEl-Moleküle in TRIS-Phosphat-Puffersystemen

Der mittlere Schmelzpunkt ($T_{m1}$) der PSMA-BiTEl-Proteindomäne mit dem niedrigsten Molekulargewicht der folgenden Formulierung wurde bestimmt:

0,2 mg/ml PSMA BiTE in 50 mM $Na_2HPO_4$, 100 mM TRIS, 0,04% Polysorbat 80, 4% Trehalose Dihydrat, pH 6,0 (mit HCl eingestellt). Mittels DSC wurde ein $T_{m1}$ von 61,1°C gemessen.

### Beispiel 8: Einfluss von Schüttelstress auf PSMA-BiTEl-Moleküle in Phosphat/TRIS Formulierungen

Generell werden BiTE-Moleküle durch Schüttelstress physikalisch destabilisiert, d.h. sie bilden Aggregate, die sich über die Dynamische Lichtstreuung (DLS) nachweisen lassen. Die Aggregatbildung findet bereits bei niedrigen BiTE-Konzentrationen von etwa 0,2 mg/ ml statt. Bei einem Proteingehalt von unter 0,2 mg/ ml adsorbieren dagegen die BiTE-Moleküle vermehrt an der Gefäßwandung.

Durch Zugabe eines Tensids (z. B. Polysorbat 20 oder 80) konnte die Adsorption der PSMA-BiTE1-Moleküle an der Gefäßwandung (z.B. von Injektionsspritzen, Infusionsbeuteln, etc.) in einigen Puffersystemen (z. B. in Phosphat- und Lysin-basierten Puffern) verhindert werden, ebenso die Aggregatbildung im Ruhezustand. Polysorbat 80 muss in einer Konzentration von mindestens 0,002% in der Zusammensetzung vorliegen, um die Adsorption der PSMA-BiTE1-Moleküle zu verhindern.

Durch Lösung der PSMA-BiTEl-Moleküle in einem Phosphatpuffer mit Tensidzusatz bei pH 6,0 ließ sich die Bildung von Aggregaten sowohl im Ruhezustand als auch während der Einwirkung von Schüttelstress verhindern. Die genannte Formulierung (Phosphatpuffer mit Tensidzusatz bei pH 6,0) war dabei hinsichtlich der Minimierung der Aggregatbildung den Formulierungen mit Lysin überlegen.

Tab. 8: PSMA-BiTEl-Aggregatbildung nach Schüttelstress; alle Proben enthalten 0,2 mg/ ml PSMA-BiTE1-Moleküle und 0,02% (m/V) Polysorbat 80

| | *pH* | Visuell | Gehalt [%] | DLS D50% [nm] |
|---|---|---|---|---|
| *$Na_2HPO_4$* | 6,0 | ok | 103,2 | 9 |
| | *6,5* | ok | 78,6 | 2451* |
| | *7,0* | ok | 52,9 | 1114* |
| | *7,5* | ok | 66,5 | 8 |
| *Lysin* | 6,5 | ok | 68,5 | 2945* |
| *$Na_2HPO_4$-Lysin-Histidin* | 6,5 | ok | 119,5 | 11 |
| *$Na_2HPO_4$-TRIS*** | 6,0 | ok | 99,0 | 6 |
| *Aggregate ** Monomer 99,6%; Dimer 0,4% | | | | |

### Beispiel 9: Konzentrationsabhängigkeit der PSMA-BiTE1-Aggregation

In Standardpuffersystemen stieg der Dimer- und Multimer-Anteil mit der Konzentration an PSMA-BiTE1-Molekülen an. Dimere und Multimere sind jedoch in Formulierungen für den therapeutischen Einsatz nur in einem geringen Rahmen akzeptabel, da sie den Wirkungsgrad der Formulierung und des therapeutischen Proteins beeinflussen und unerwünschte immunologische Effekte provozieren können. Typischerweise begrenzt man die Dimere auf einen Wert von max. 5% und versucht, diesen Wert möglichst zu unterschreiten. Multimere und Low Molecular Weight (LMW)-Fragmente sollen ebenfalls minimiert werden, bzw. gar nicht vorhanden sein. Das Monomer/Dimer Verhältnis wird, ebenso wie der Anteil an Multimeren und Low Molecular Weight-Fragmenten mit Size Exclusion Chromatographie (SEC) gemessen.

Mit dem Puffersystem umfassend 50 mM $Na_2HPO_4$ und 100 mM TRIS bei pH 6,0 konnte die Dimer- und Multimerbildung unter den PSMA-BiTEl-Molekülen während der Aufkonzentrierung ausreichend reduziert werden.

Mit Hilfe dieses Puffersystems war die Herstellung einer stabilen BiTE-Formulierung mit einem Gehalt von >2 mg/ml möglich (Tabelle 9).

*Tab. 9:* PSMA-BiTE1-*Moleküle in 50 mM $Na_2HPO_4$ und 100mM TRIS bei pH 6,0*

| Protein-Gehalt [mg/ml] | 0,321 | 0,333 | 0,530 | 0,883 | 1,308 | 1,330 | 2,138 | 3,228 |
|---|---|---|---|---|---|---|---|---|
| Monomer [%] | 98.17 | 98.30 | 97.89 | 95.15 | 98.33 | 95.95 | 96.28 | 96.97 |
| Dimer [%] | 0.58 | 0.42 | 1.18 | 1.71 | 1.14 | 1.92 | 2.26 | 2.03 |
| Multimer [%] | < 0,05 | < 0,05 | < 0,05 | < 0,05 | < 0,05 | < 0,05 | < 0,05 | < 0,05 |
| LMW [%] | 1.24 | 1.28 | 0.93 | 3.14 | 0.53 | 2.14 | 1.45 | 1.00 |
| LMW = Low Molecular Weight-Fragmente | | | | | | | | |

**Beispiel 10: Einfluss von Trehalose und Polysorbat auf die PSMA-BiTE1-Stabilität**

Die Zugabe von Trehalose und Polysorbat führte nicht zu einer erhöhten Bildung von Dimeren, Multimeren, oder LMW-Fragmenten (Tabelle 10).

*Tab. 10:* PSMA-BiTE1-*Moleküle in 50 mM $Na_2HPO_4$ und 100mM TRIS bei pH 6,0 mit/ ohne Trehalose und Polysorbat 80*

| *Polysorbat 80 (% m/V)* | - | *0.04%* | - | *0.04%* |
|---|---|---|---|---|
| *Trehalose (% m/V)* | - | - | *4%* | *4%* |
| Protein-Gehalt [mg/ ml] | 1,209 | 1,235 | 1,191 | 1,217 |
| SEC Monomer [%] | 98,3 | 98,3 | 98,5 | 98,2 |
| Dimer [%] | 1,6 | 1,7 | 1,5 | 1,7 |
| Multimer [%] | 0,1 | 0,1 | < 0,05 | 0,1 |
| LMW [%] | < 0,05 | < 0,05 | < 0,05 | < 0,05 |

**Beispiel 11: Lagerstabilität der PSMA-BiTE1-Moleküle**

Die Lagerstabilität der PSMA-BiTEl-Moleküle lässt sich anhand der Zunahme des Anteils an Dimeren und/oder Multimeren in Abhängigkeit von der Lagerzeit bestimmen. Je schneller der Anteil dieser Aggregate ansteigt, desto geringer ist die Lagerstabilität.

Experimentell konnte gezeigt werden, dass die PSMA-BiTE1-Moleküle in einer Konzentration von 90 µg/ml, 500µg/ml und 2 mg/ml für einen Zeitraum von 9 Tagen gegenüber Di- und/oder Multimerbildung stabil waren (Tabelle 11). Die Formulierungen wurden in Injektionsspritzen bei etwa 2-8°C aufbewahrt, nach einer initialen Phase von 4 bis 16 Stunden bei Raumtemperatur (etwa 20°C). Die Zusammensetzungen enthielten neben den PSMA-BiTEl-Molekülen 50 mM $Na_2HPO_4$, 100 mM TRIS, 0,04% Polysorbat 80 und 4% Trehalose. Der Anteil an PSMA-BiTEl-Monomeren wurde mittels SEC-HPLC gemessen und mit dem PSMA-BiTEl-Monomeranteil zu Beginn der Versuche (d.h. am Tag 0) verglichen. Diese relative Reinheit betrug nach 9 Tagen bei Zusammensetzungen mit einer PSMA-BiTEl-Ausgangskonzentration von 90 µg/ml und 500µg/ml 100%, d.h. der Anteil der Monomere war über diese Zeit nicht gesunken. Die relative Reinheit bei den Zusammensetzungen mit 2 mg/ml PSMA-BiTEl-Molekülen betrug nach 9 Tagen 97%, was einer Abnahme der Monomere um etwa 3% absolut entspricht (von 97,58% am Tag 0 auf 94,81% am Tag 9).

Tab. 11/1: Bestimmung der Reinheit (d.h. des Monomeranteils) der PSMA-BiTE-Moleküle mittels SEC-HPLC über einen Zeitraum von 9 Tagen

| Tag | PSMA-BiTE1-Konzentration [µg/ml] | Reinheit, Monomeranteil [%] | | | | | | Relative Reinheit (Monomeranteil) im Vergleich zum T0 Wert | | | |
|---|---|---|---|---|---|---|---|---|---|---|---|
| | | Test 1 | Test 2 | Test 3 | Mittel | Standardabweichung | VK [%] | Test 1 | Test 2 | Test 3 | Mittel |
| 0 | 90 | 98,30 | 98,21 | 98,20 | **98,24** | 0,06 | 0,1 | - | - | - | - |
| 2 | | 98,35 | 98,39 | 98,37 | **98,37** | 0,02 | 0,0 | 100 | 100 | 100 | 100 |
| 7 | | 98,59 | 98,53 | 98,40 | **98,51** | 0,10 | 0,1 | 100 | 100 | 100 | 100 |
| 9 | | 98,37 | 98,36 | 98,39 | **98,37** | 0,02 | 0,0 | 100 | 100 | 100 | 100 |
| 0 | 500 | 97,93 | 97,95 | 98,01 | **97,96** | 0,04 | 0,0 | - | - | - | - |
| 2 | | 98,03 | 98,07 | 97,77 | **97,96** | 0,16 | 0,2 | 100 | 100 | 100 | 100 |
| 7 | | 97,90 | 97,67 | 97,91 | **97,83** | 0,14 | 0,1 | 100 | 100 | 100 | 100 |
| 9 | | 97,71 | 97,66 | 97,87 | **97,75** | 0,11 | 0,1 | 100 | 100 | 100 | 100 |
| 0 | 2000 | 97,45 | 97,54 | 97,75 | **97,58** | 0,15 | 0,2 | - | - | - | - |
| 2 | | 96,61 | 96,69 | 96,57 | **96,62** | 0,06 | 0,1 | 99 | 99 | 99 | 99 |
| 7 | | 94,79 | 95,34 | 95,04 | **95,06** | 0,28 | 0,3 | 97 | 98 | 97 | 97 |
| 9 | | 94,71 | 94,88 | 94,84 | **94,81** | 0,09 | 0,1 | 97 | 97 | 97 | 97 |

In anderen Versuchen zeigte sich in der flüssigen Formulierung mit einer PSMA- BiTE1-Konzentration von 2 mg/ml bei 2-8°C im Verlauf einer Woche ein moderater Anstieg des Dimer-Anteils um 2,5% (von 3% auf 5,5%), bei gleichzeitig stabilem Multimeranteil (Tabelle 12). Bei dieser Konzentration sind die PSMA-BiTEl-Moleküle damit ausreichend lange stabil, um annähernd verlustfreie Abfüllung und annähernd verlustfreien Gebrauch zu gewährleisten.

Für eine Langzeitlagerung (d.h. die Lagerung für einen Zeitraum erheblich länger als eine Woche) können PSMA-BiTE1-Lösungen zur Gewährleistung ihrer Stabilität jedoch entweder eingefroren (-80°C) oder lyophilisert werden. Eine Lyophilisation der PSMA-BITE1-enthaltenden Formulierungen war unter Erhalt der Bioaktivität war möglich, wie in Beispiel 17 gezeigt.

*Tab. 12: Lagerstabilität der BiTE-Formulierung bei 2-8°C (PSMA-BiTE1-Konzentration 2mg/ml)*

| Lagerzeit [Tage] | SEC Monomer [%] | SEC Dimer[%] | SEC Multimer [%] |
|---|---|---|---|
| Start | 96,4 | 3,0 | 0,6 |
| 0,25 | 96,2 | 3,2 | 0,6 |
| 0,5 | 96,0 | 3,4 | 0,6 |
| 0,75 | 95,9 | 3,6 | 0,5 |
| 1 | 95,8 | 3,7 | 0,5 |
| 3 | 95,7 | 4,0 | 0,4 |
| 7 | 94,2 | 5,5 | 0,4 |
| 15 | 93,7 | 6,1 | 0,2 |
| 28 | 92,6 | 7,2 | 0,2 |
| 65 | 90,7 | 9,2 | 0,2 |
| 94 | 90,3 | 9,5 | 0,2 |
| 161 | 88,2 | 11,3 | 0,5 |
| 251 | 87,8 | 11,7 | 0,5 |

**Beispiel 12: Einfluss des pH-Wertes auf die PSMA-BiTE1-Stabilität**

Grundsätzlich sind PSMA-BiTEl-Moleküle in der gewählten Formulierung mit TRIS, Phosphat (hier: $Na_2HPO_4$), Trehalose und Polysorbat in einem pH-Wertbereich zwischen pH 5,0 und 7,5 stabil. Bei einem pH-Wert oberhalb pH 6) nimmt allerdings der Dimeranteil (>2%) nach dem Scherstress zu (Tabelle 13).

*Tab. 13: 2 mg PSMA-BiTE1-Moleküle pro ml in 50mM $Na_2HPO_4$, 100mM TRIS pH [Variabel]; 4% (m/V)Trehalose, 0,04% (% m/V) Polysorbat 80*

| pH | 5,0 | 5,5 | 6,0 | 6,5 | 7,0 | 7,5 |
|---|---|---|---|---|---|---|
| *Nach Herstellung* | | | | | | |
| Protein-Gehalt [mg/ ml] | 2,01 | 2,02 | 2,11 | 1,90 | 2,13 | 2,20 |
| DLS (D50%) [nm] | 10 | 11 | 15 | 11 | 12 | 10 |
| SEC Monomer [%] | 98,62 | 97,88 | 98,48 | 97,74 | 98,58 | 98,53 |
| Dimer [%] | 1,22 | 1,81 | 1,36 | 1,96 | 1,42 | 1,47 |
| Multimer [%] | < 0,05 | < 0,05 | < 0,05 | < 0,05 | < 0,05 | < 0,05 |
| LMW [%] | 0,15 | 0,31 | 0,16 | 0,30 | < 0,05 | < 0,05 |

(fortgesetzt)

| Nach Scherstress | | | | | | |
|---|---|---|---|---|---|
| Protein-Gehalt [mg/ ml] | 2,00 | 2,01 | 2,11 | 1,90 | 2,13 | 2,20 |
| Protein-Gehalt [%] | 99,5 | 99,6 | 99,8 | 100,1 | 99,9 | 100,1 |
| DLS (D50%) [nm] | 10 | 15 | 12 | 10 | - | - |
| SEC Monomer [%] | 98,37 | 98,21 | 98,23 | 97,83 | 97,42 | 97,53 |
| Dimer [%] | 1,63 | 1,79 | 1,77 | 2,17 | 2,58 | 2,47 |
| Multimer [%] | < 0,05 | < 0,05 | < 0,05 | < 0,05 | < 0,05 | < 0,05 |
| LMW [%] | < 0,05 | < 0,05 | < 0,05 | < 0,05 | < 0,05 | < 0,05 |

**Beispiel 13: Einfluss von TRIS und Phosphat auf die PSMA-BiTE1-Stabilität**

In Untersuchungen hinsichtlich unterschiedlicher Pufferstärken in der Formulierung wurden gezeigt, dass die Pufferstärken in der Formulierung variiert werden können: einsetzbar hinsichtlich der Minimierung der PSMA-BiTEl-Dimer-Bildung sind 20 bis 100 mM $Na_2HPO_4$ und 50 bis 200mM TRIS bei pH 6,0. Alle Kombinationen erlauben ein Aufkonzentrieren und die Einwirkung von Scherstress.

Ohne TRIS ließen sich die PSMA-BiTEl-Moleküle nicht aufkonzentrieren und ohne $Na_2HPO_4$ stieg der Dimeranteil auf über >2% nach Einwirkung von Scherstress. Außerdem zeigte der Phosphatpuffer eine gute Pufferwirkung bei pH 6,0 und unterstützt die Thermostabilität der PSMA-BiTE1-Moleküle.

Tab. 14: 2 mg/ml PSMA-BiTEl-Moleküle in [Variabel] $Na_2HPO_4$, [Variabel] TRIS pH 6,0; 4% (m/V) Trehalose, 0,04% (m/V) Polysorbat 80

| $Na_2HPO_4$ [mM] | - | 20 | 50 | 50 | 50 | 100 |
|---|---|---|---|---|---|---|
| TRIS [mM] | 100 | 100 | 100 | 50 | 200 | 100 |
| Nach Herstellung | | | | | | |
| Protein-Gehalt [mg/ ml] | 2,23 | 2,08 | 2,11 | 2,19 | 2,17 | 2,07 |
| DLS (D50%) [nm] | 11 | 9 | 15 | 12 | 15 | 12 |
| SEC Monomer [%] | 98,11 | 98,78 | 98,48 | 99,22 | 99,01 | 99,19 |
| Dimer [%] | 1,57 | 1,22 | 1,36 | 0,78 | 0,99 | 0,81 |
| Multimer [%] | < 0,05 | < 0,05 | < 0,05 | < 0,05 | < 0,05 | < 0,05 |
| LMW [%] | 0,31 | < 0,05 | 0,16 | < 0,05 | < 0,05 | 0,30 |
| Nach Scherstress | | | | | | |
| Protein-Gehalt [mg/ ml] | 2,22 | 2,08 | 2,11 | 2,20 | 2,16 | 2,08 |
| Protein-Gehalt [%] | 99,9 | 100,0 | 99,8 | 100,4 | 99,9 | 100,1 |
| DLS (D50%) [nm] | - | - | 12 | 13 | 13 | 12 |
| SEC Monomer [%] | 97,90 | 98,26 | 98,23 | 98,55 | 98,32 | 98,60 |

(fortgesetzt)

| Nach Scherstress | | | | | | |
|---|---|---|---|---|---|---|
| Dimer [%] | 2,10 | 1,73 | 1,77 | 1,45 | 1,68 | 1,40 |
| Multimer [%] | < 0,05 | < 0,05 | < 0,05 | < 0,05 | < 0,05 | < 0,05 |
| LMW [%] | < 0,05 | < 0,05 | < 0,05 | < 0,05 | < 0,05 | < 0,05 |

**Beispiel 14**: **Einfluss verschiedener Netzmittel auf die PSMA-BiTEI-Stabilität**

Verschiedene Polysorbate können die PSMA-BiTEI-Moleküle in der Formulierung gegen Scherstress stabilisieren. Mit Polysorbat 80 wurden jedoch die besten Ergebnisse erzielt. Andere Stabilisatoren wie z.B. Synperonic F68 hatten ebenfalls positive Effekte.

0,04% bis 0,10% *(m/V)* Polysorbat 80 hatte eine gute stabilisierende Wirkung auf die PSMA-BiTE1-Moleküle während des Scherstresses. 0,004% *(m/V)* Polysorbat waren hier nicht ausreichend, um eine inakzeptable Zunahme der Dimerbildung nach Einwirkung von Scherstress zu verhindern.

*Tab. 15: 2 mg/ml* PSMA-BiTEI-Moleküle *in 50mM* $Na_2HPO_4$*, 100mM TRIS pH 6,0; 4% (m/V) Trehalose, [Variabel] Netzmittel*

| Netzmittel | Polysorbat 80 | Polysorbat 20 | Synperonic F68 | Polysorbat 80 | Polysorbat 80 |
|---|---|---|---|---|---|
| Netzmittel [% (m/V)] | 0,04 | 0,04 | 0,04 | 0,004 | 0,10 |
| **Nach Herstellung** | | | | | |
| Protein-Gehalt [mg/ml] | 2,11 | 2,13 | 2,10 | 2,13 | 2,08 |
| DLS (D50%) [nm] | 15 | 12 | 10 | 13 | 14 |
| SEC Monomer [%] | 98,48 | 97,92 | 98,59 | 98,47 | 98,36 |
| Dimer [%] | 1,36 | 2,08 | 1,24 | 1,36 | 1,46 |
| Multimer [%] | < 0,05 | < 0,05 | < 0,05 | < 0,05 | < 0,05 |
| LMW [%] | 0,16 | < 0,05 | 0,16 | 0,17 | 0,17 |
| **Nach Scherstress** | | | | | |
| Protein-Gehalt [mg/ml] | 2,11 | 2,11 | 2,10 | 1,98 | 2,07 |
| Protein-Gehalt [%] | 99,8 | 99,2 | 99,9 | 93,3 | 99,5 |
| DLS (D50%) [nm] | 12 | 14 | 10 | 3139 | 15 |
| SEC Monomer [%] | 98,23 | 96,54 | - | 92,54 | 98,09 |
| Dimer [%] | 1,77 | 3,46 | - | 7,46 | 1,91 |
| Multimer [%] | < 0,05 | < 0,05 | - | < 0,05 | < 0,05 |

(fortgesetzt)

| | | | | | |
|---|---|---|---|---|---|
| **Nach Scherstress** | | | | | |
| LMW [%] | < 0,05 | < 0,05 | - | < 0,05 | < 0,05 |

**Beispiel 15: Einfluss der PSMA-BiTEl-Konzentration auf die Stabilität der Formulierung**

Mit der Formulierung 50mM $Na_2HPO_4$, 100mM TRIS pH 6,0; 4% (m/V) Trehalose, 0,04% (m/V) Polysorbat 80 ließen sich PSMA-BiTE1 Konzentrationen bis 2 mg/ ml herstellen. Bei höheren PSMA-BiTE1 Konzentrationen stieg der Dimeranteil deutlich an. Inakzeptable Werte wurden jedoch erst bei PSMA-BiTE-Konzentrationen von mehr als 4 mg/ml und nach der Einwirkung von Scherkräften gemessen (9,25% Dimere bei einer PSMA-BiTE-Konzentration von etwa 11,2 mg/ml).

*Tab. 16: [Variabel] mg/ml PSMA-BiTEl-Moleküle in 50mM $Na_2HPO_4$, 100mM TRIS pH 6,0; 4% (m/V) Trehalose, 0,04% (m/V) Polysorbat 80*

| PSMA-BiTE1 Konz. [mg/ml] | 0,4 | 2 | 4 | 11 |
|---|---|---|---|---|
| **Nach Herstellung** | | | | |
| Protein-Gehalt [mg/ ml] | 0,44 | 2,11 | 4,42 | 11,17 |
| DLS (D50%) [nm] | 12 | 15 | 17 | 18 |
| SEC Monomer [%] | 98,67 | 98,48 | 96,62 | 95,63 |
| Dimer [%] | 1,16 | 1,36 | 2,82 | 3,73 |
| Multimer [%] | < 0,05 | < 0,05 | < 0,05 | < 0,05 |
| LMW [%] | 0,16 | 0,16 | 0,56 | 0,63 |
| **Nach Scherstress** | | | | |
| Protein-Gehalt [mg/ ml] | 0,44 | 2,11 | 4,39 | 11,32 |
| Protein-Gehalt [%] | 99,8 | 99,8 | 99,2 | 101,3 |
| DLS (D50%) [nm] | 10 | 12 | 12 | 16 |
| SEC Monomer [%] | 99,53 | 98,23 | 96,32 | 90,75 |
| Dimer [%] | 0,47 | 1,77 | 3,68 | 9,25 |
| Multimer [%] | < 0,05 | < 0,05 | < 0,05 | < 0,05 |
| LMW [%] | < 0,05 | < 0,05 | < 0,05 | < 0,05 |

**Beispiel 16: Lyophilisierung**

Nach der Fertigstellung der PSMA-BiTEl-Zusammensetzung wurde diese Lyophilisiert. Hierfür stehen zahlreiche Gefriertrocknungsanlagen zur Verfügung, wie z.B. der Genesis Super XL von SP Scientific. Gefriertrocknung wird erreicht durch das Einfrieren einer Substanz und die anschließende Sublimation des Eises ohne das Durchlaufen einer flüssigen Phase.

*Tab. 17: Programm zur Gefriertrocknung von **PSMA-BiTE** Formulierungen (Gesamtdauer: 42 h).*

| | Ts [°C] | t [min] | Vakuum [$\mu$bar] | Rampe/ Halten |
|---|---|---|---|---|
| **Einfrierphase** | | | | |
| 1 | Raumtemper | 0 | - | Halten |
| 2 | -45 | 30 | - | Rampe |
| 3 | -45 | 240 | - | Halten |

(fortgesetzt)

| Haupttrocknung | | | | |
|---|---|---|---|---|
| 1 | -20 | 60 | 100 | Rampe |
| 2 | -20 | 1000 | 100 | Halten |
| Nachtrocknung | | | | |
| 1 | 25 | 60 | 10 | Rampe |
| 2 | 25 | 1140 | 10 | Halten |
| "Rampe" = kontinuierliche Temperatur erhöhung oder -Senkung | | | | |

In der Einfrierphase wurde das Produkt in einer "Rampe", d.h. kontinuierlich, innerhalb von 30 min. von Raumtemperatur auf -45°C abgekühlt. Damit die Produktlösung vollständig einfriert, wurde diese Temperatur für 240 min. gehalten.

Danach erfolgte die Haupttrocknungsphase. Bei einem Kammervakuum von 100 μbar wurde die Zusammensetzung innerhalb von 60 min. auf -20°C erwärmt. Diese Temperatur wird für 1000 min. gehalten; dann war die Haupttrocknung abgeschlossen. Für die anschließende Nachtrocknung wurde die Zusammensetzung bei einem Vakuum von 10 μbar auf 25°C erwärmt. Diese Bedingungen wurden für 1140 min. gehalten, um das Restwasser bis auf $\leq$ 2% (Nachweis mittels Karl-Fischer-Titration) zu entfernen.

Am Ende des Trocknungsprozess wurde die Anlage belüftet und die Lyophilisationsgefäße verschlossen.

**Beispiel 17: Bioaktivität von PSMA-BiTEl-Lyophilisaten nach Langzeitlagerung und Rekonstituierung**

Zusammensetzungen mit PSMA-BiTEl-Molekülen wurden als Lyophilisat bis zu 12 Monate bei 2-8°C und bei 25°C/60% relativer Luftfeuchte gelagert. Nach 6 und 12 Monaten wurde jeweils Lösung aus Lyophilisat rekonstituiert und im zellbasierten Aktivitätstest analysiert. Die Messungen (mittels CytoTox-Glo Cytotoxicity Assay von Promega) ergaben eine unveränderte Bioaktivität, sowohl nach 6 als auch nach 12-monatiger Lagerung unter den genannten Bedingungen.

Darüber hinaus wurde die Lagerstabilität der rekonstituierten PSMA-BiTE1-Lösung analysiert. Die Lösung wurde nach Rekonstituierung zunächst 7 Tage im Kühlschrank (2-8°C) gelagert und anschließend 16 Stunden bei Raumtemperatur (+20 $\pm$ 5°C). Anschließend wurde auch hier die Bioaktivität der PSMA-BiTEl-Moleküle mittels zellbasiertem Aktivitätstest ermittelt. Sie betrug in rekonstituierter Lösung nach der genannten weiteren Lagerung 96%.

Die Bioaktivität der PSMA-BiTEl-Moleküle in der lyophilisierten Formulierung war folglich nach einer Lagerung über 6-12 Monate unter den genannten Lagerbedingungen stabil. Gleiches gilt für die aus diesem Lyophilisat rekonstituierte Lösung nach einer Lagerung für 7 Tage bei 2-8°C und 16 Stunden bei Raumtemperatur.

**Beispiel 18: Einfluss von Scherstress bei der Applikation mittels Injektionsspritze und Kanüle auf die PSMA-BiTEl-Dimerbildung**

Zusammensetzung: 2,17 mg/ml PSMA-BiTEl-Moleküle, 50mM $Na_2HPO_4$, 100mM TRIS pH 6,0; 4% Trehalose, 0,04% Polysorbat 80

Material: Einmalspritzen (BD 2ml), Kanülen (Braun Sterican 0,70x30mm Gr.12; 22G) und Braunglas Vials (6R) und Cryotubes.

Durchführung:

30 Vials wurden aufgetaut. 6 Vials wurden als Startwerte verwendet. Für jeden Versuch wurden 6 Vials verwendet, d.h. mit der Spritze/Kanüle entnommen und alle in ein Braunglas bzw. Cryotube injiziert.

Versuche:

1. Langsame Injektion der PSMA-BiTEl-Zusammensetzung in ein CryoTube (SpL Cryo)

2. Langsame Injektion der PSMA-BiTEl-Zusammensetzung in ein Braunglas (SpL Glas)

3. Schnelle Injektion der PSMA-BiTEl-Zusammensetzung in ein CryoTube (SpL Cryo)

4. Schnelle Injektion der PSMA-BiTEl-Zusammensetzung in ein Braunglas (SpL Glas)

Tab. 18: PSMA-BiTEl-Dimerbildung nach Einwirkung von Scherstress durch Injektion

|  | Start | SpL Cryo | SpL Glas | SpS Cryo | SpS Glas |
|---|---|---|---|---|---|
| PSMA-BiTE1- | 2,17 | 2,21 | 2,16 | 2,19 | 2,16 |
| SEC Monomer [%] | 96,6 | 96,5 | 96,4 | 96,4 | 96,4 |
| SEC Dimer [%] | 3,3 | 3,4 | 3,5 | 3,4 | 3,4 |
| SEC Multimere | 0,1 | 0,1 | 0,1 | 0,1 | 0,1 |

Gemessen wurde die Dimerbildung mittels Differential Scanning Fluorimetry (DSF). Ein signifikanter Anstieg des Dimeranteils nach der Injektion der Zusammensetzung mittels Injektionsspritzen und Kanülen war nicht zu verzeichnen. Dieses Ergebnis zeigt, dass die erfindungsgemäße Formulierung in allen Fällen stabil gegenüber der Einwirkung der erzeugten Scherkräfte ist.

**Beispiel 19: Bioverfügbarkeit von PSMA-BiTEl nach subkutaner Applikation**

Mit der Formulierung 50mM $Na_2HPO_4$, 100mM TRIS pH 6,0; 4% Trehalose, 0,04% Polysorbat 80 war es möglich, eine Bioverfügbarkeit nach subkutaner Applikation von 66% zu erzielen. Die s.c. Bioverfügbarkeit der PSMA-BiITE1-Moleküle wurde in 4 weiblichen *Cynomolgus* Affen untersucht. Die Dosierung betrug 45 µg/kg bei der s.c. Anwendung und wurde mit einer i.v. Anwendung von 5 und 15 µg/kg Körpergewicht (BW) verglichen. Die Stammlösung mit 2 mg/mL wurde mit physiologischer Kochsalzlösung verdünnt. Die Infusionsdauer der i.v. Anwendung betrug 1 Stunde und die Infusionsrate betrug 1ml/ kg BW. Als Injektionsort wurde eine oberflächliche Vene gewählt (*V. saphena parva*). Bei der s.c. Anwendung wurde die Testlösung mit 0.15 mL/ kg BW in den lateralen Brustbereich injiziert. Die Blutspiegel wurden mit einem ELISA Test untersucht. Dazu wurde die ECL Technologie eingesetzt. Die untere Quantifizierungsgrenze (LLOQ) der Methode lag bei 4µg/L.

Methoden

**Herstellung der PSMA-BiTE-Moleküle**

Herstellungsverfahren für BiTE Moleküle insbesondere für PSMA-BiTE Moleküle sind z.B. in WO2010037836 A2 beschrieben.

Zunächst wurde das PSMA-BiTE1 kodierende rekombinante BiTE-DNA-Konstrukt in einen geeigneten Expressionsvektor integriert und mit diesem stabil in eukaryotische CHO (Chinese Hamster Ovary)-Zellen eingebracht. Die transfizierten CHO-Zellen wurden in einem Bioreaktor mit einem geeigneten Nährmedium kultiviert und das sezernierte Protein durch Filtration von den Zellen isoliert. Die Aufreinigung der BiTE-Moleküle umfasste einen Austausch der Puffersubstanzen gegen TRIS und Phosphat mittels Größenausschluss-Chromatographie (SEC) und die anschließende Aufkonzentrierung mittels Ultrafiltration und Diafiltration. Zusätzlich wurde ein Polyol (bevorzugt Trehalose) und ein Netzmittel (bevorzugt Polysorbat 80) hinzugegeben. Gelagert wurde die Zusammensetzung bei unter -60°C.

**Differential-Scanning-Fluorimetrie (DSF)**

Die Messungen zur Stabilität der PSMA-BiTEl-Moleküle (z.B. nach Scherstress) wurden mit einem 750 Fast Real Time PCR System (Applied Biosystems) durchgeführt. Unterschiedliche PSMA-BiTEl-Konzentrationen (zwischen 0,15 und 0,005 mg/ ml) wurden in 96 well Platten (Mikrotiterplatten) mit einem fluoreszierenden Farbstoff (z.B. "Sypro® Orange 5000") versetzt und in einem PCR System (750 Fast Real Time PCR System, Applied Biosystems) vermessen. Die Temperatur wurde von 20°C auf 90°C erhöht. Die Schmelzpunkte der Proteine wurden über eine Fluoreszenzdetektion ermittelt, die sich temperaturabhängig ergibt, wenn der fluoreszierende Farbstoff mit den hydrophoben Regionen des Proteins reagiert.

**Dynamische Differenzkalorimetrie (Differential Scanning Calorimetry, DSC)**

Ermittelt wurde die thermische Auffaltungstemperatur ($T_m$) der PSMA-BiTEl-Moleküle mittels DSC. Die Proben wurden hierfür von 20°C auf 105°C erwärmt und der Schmelzpunkt der Polypeptide mit einem Kalorimeter ermittelt. Es wurde ein VP-DSC Gerät von GE Healthcare eingesetzt.

**Schüttelstress**

Die Proben wurden gestresst, indem sie auf einem Laborschüttler (IKA, HS 260) in einer Temperierten Kammer (MMM, FrioCell 200) geschüttelt wurden. Der kritische Quality Parameter Aggregation wurde nach 24 Stunden bei 300 rpm und 20°C ermittelt.

**Optische Kontrolle**

Die Stabilität PSMA BITE Lösungen wurde nach dem Scherstress optisch kontrolliert, indem die Lösungen gegen einen dunklen Hintergrund gehalten und auf sichtbare Partikel oder Trübungen untersucht wurden. Eine klare Lösung nach Schüttelstresstest deutet auf eine geringe bis abwesende Dimer- und/oder Multimerbildung hin, während eine sichtbare Trübung der Lösung mit einem hohen Anteil an Dimeren und/oder Multimeren korreliert.

**Dynamische Lichtstreuung (DLS)**

Die dynamische Lichtstreuung ist eine Methode zur Analyse des Streulichtes eines Lasers an einer gelösten bzw. suspendierten Probe. Gemessen wird dabei der hydrodynamische Radius, der wiederum Rückschlüsse auf den Aggregierungszustand der PSMA-BiTEl-Moleküle zulässt. Der hydrodynamische Radius wurde mit einem Horiba LB 550 (Retsch Technology) gemessen.

**Bestimmung des Proteingehaltes**

Der Proteingehalt wurde spektrometrisch mit einem Nanodrop 2000 (Thermo Scientific) bei 280 nm gemessen. Alle Proben wurden gegen die entsprechenden Placebo- bzw. Pufferlösungen vermessen.
Jede Probelösung wurde 3x in den Messbereich des Nanodrop Gerätes pipettiert ($2\mu$l) und jeweils doppelt vermessen, danach wurden diese Messwerte (n=6) gemittelt. Der Proteingehalt [mg/ml] wurde über eine zuvor erstellte Kalibrierfunktion (Abhängigkeit Absorption zu Proteingehalt) des Proteins aus den gemittelten Messwerten errechnet.

**Elektrochemilumineszenz-Messung (ECL Assay)**

Für diese Methode wurden SULFO-TAG™ Markierungen verwendet, die nach elektrochemischer Stimulation Licht emittieren. Die Stimulation erfolgte an den Oberflächen von Elektroden sogenannter MULTI-ARRAY Mikroplatten. Das emittierte Licht wurde bei ca. 620nm mit einem Detektor gemessen.
Die Messung basierte auf der "Sandwich"-Methode, bei der PSMA-BiTEl-Moleküle in Lösung mittels polyklonaler goat anti-PSMA-BiTEl-Antikörper auf einer Mikroplatte immobilisiert wurden. Penta-His-Biotin wurde dann an die immobilisierten BiTE-Moleküle gebunden und mittels SULFO-TAG™ -konjugiertem Streptavidin nachgewiesen. Die Proben wurden in einem Sektor Imager 2400 ausgelesen.

**Größenausschluss-Chromatographie (size exclusion chromatography, SEC)**

Zur Bestimmung der Anteile an Monomeren und Dimeren sowie der Low Molecular Weight (LMW)- und High Molecular Weight (HMW)-Anteile wurde eine Größenausschluss-Chromatographie mithilfe einer HPLC Anlage durchgeführt. Die Messung erfolgte mit einem Fluoreszenzdetektor und die Berechnung erfolgte mittels Flächenprozentmethode. Als Säule wurde eine Standardsäule für die SEC von Proteinen verwendet, z.B. eine Tosoh Biosep TSK gel G3000 SWXL 5 $\mu$m, 300mm, Length x 7.8mm i.D. oder ein gleichwertiges Material.

**Bestimmung von PSMA-BITEl-Konzentrationen im Serum nach i.v. und s.c. Gabe**

Die Konzentration von PSMA-BITE1 im Serum von Cynamolgus Affen wurde mittels ELISA gemessen. Die Detektion erfolgte über Elektrochemolumineszenz. Die Detektionsgrenze (LLOQ) lag bei 0,98 $\mu$g/L, bei einer Präzision (Precision) von 3 bis 28% und einer Genauigkeit (Accuracy) von 70 bis 100%.

**Zellbasierter Aktivitätstest**

Der zellbasierte Zytotoxizitätsassay dient als Routineassay zur Bestimmung der relativen Aktivität von PSMA-BiTEl-Proben. Durch die bispezifische Bindung von PSMA-BiTE1 an human/cynomolgus CD3- und human/cynomolgus PSMA-positiven Zellen erfolgt nach Aktivierung der T-Zellen (Effektorzellen) die T-zellvermittelte Lyse der Zielzellen.

Der Nachweis der Zytotoxizität erfolgt mittels des lumineszenten CytoTox-Glo Cytotoxicity Assays von Promega. Als Messgröße dient hierbei die Menge des freigesetzten Lichtsignals, das mit der Anzahl sterbender Zellen korreliert. Weitere Aktivitätstests sind in WO2010037836 A2 beschrieben.

**Zellbasierter Zytotoxizitätstest zur Bestimmung der relativen Aktivität der PSMA-BiTEl - Proben**

| Geräte und Material | 1. Sterilwerkbank |
| | 2. Zellinkubator |
| | 3. Mikroskop |
| | 4. Zellzählgerät z.B. Hemozytometer |
| | 5. 96-well U-Boden-Platten, klar (z.B. Greiner Bio-one) |
| | 6. 96-well Flachbodenplatten, weiß (z.B. Nunc, 3058078) |
| | 7. Zellkultur-Flaschen |
| | 8. Multiplattenmessgerät |
| | |
| Reagenzien | 1. Effektor Zellen z.B. MC15 |
| | 2. Zielzellen z.B. C4-2 |
| | 3. 0.4 % Trypan - Blau |
| | 4. Penicillin - Streptomycin |
| | 5. L-Glutamin (200 mM) |
| | 6. Interleukin 2 |
| | 7. Medium - MC15: Advanced RPMI 1640 |
| | 8. fetales Kälberserum (FKS), z.B. Gibco 10270106 |
| | 9. PBS, z.B. Gibco 20012 |
| | 10. Medium - C4-2: RPMI 1640 mit L-Glutamine, z.B. Gibco 11835063 |
| | 11. Nachweisreagenz z.B. CytoTox Glo, Promega G9291 |
| | |
| Wachstumsmedien | Medium für MC 15 Zellen z.B. |
| | 900 ml Advanced RPMI 1640 |
| | +100 ml FKS |
| | +10 ml Penicillin - Streptomycin |
| | +10 ml L-Glutamin (200mM) |
| | +10 - 20 $\mu$l [100-200 U/ml] Interleukin 2 |
| | Medium für C4 - 2 Zellen z.B. |
| | 900 ml RPMI 1640 mit L-Glutamin |
| | +100 ml FKS |
| | +10 ml Penicillin Streptomycin |
| Assaymedium | Assaymedium, z.B. |
| | 900 ml RPMI 1640 mit L-Glutamine |
| | +100 ml FKS |
| | |
| Beginn der Zellkultur MC 15 | Zellen für den Assay wurden in flüssigem Stickstoff aufbewahrt. Das Auftauen der Zellen erfolgte schnell bei 37 °C. Dann wurden die Zellen in 15 ml kaltem Medium resuspendiert und für 10 min. inkubiert. Dann wurden die Zellen für z.B. 7 Minuten bei 700g zentrifugiert, der Überstand verworfen und das Pellet in 10 ml Wachstumsmedium resuspendiert. |

(fortgesetzt)

Zellen wurden bei 37 °C und 5 % $CO_2$ für ca. 3-4 Tage inkubiert.

| | |
|---|---|
| Subkultivierung MC 15 | Aliquote von lebenden Zellen aus der Kultur werden gezählt |
| | Zentrifugieren für 7 Minuten bei 170g |
| | Überstand verwerfen und das Pellet mit Wachstumsmedium auf eine Konzentration von 0,5 x$10^6$ bis 1,5 x $10^6$ Zellen/ml einstellen. Die Zellen sollten 2-3 mal in der Woche passagiert werden. |
| Beginn der Zellkultur C4 -2 | Zellen für den Assay werden in flüssigem Stickstoff aufbewahrt. Das Auftauen der Zellen erfolgt schnell bei 37 °C. Dann werden die Zellen in 20 ml Medium resuspendiert. Dann für z. B. 7 Minuten bei 700g zentrifugieren. Überstand verwerfen und das Pellet in 10 ml Wachstumsmedium resuspendieren. |
| | Zellen werden bei 37 °C und 5 % $CO_2$ für ca. 3-4 Tage inkubiert. |
| Subkultivierung C4-2 | Medium aus der Flasche entfernen und verwerfen |
| | Zellschicht vorsichtig mit 10ml PBS spülen |
| | 2-3 ml Trypsin hinzufügen und bei 37 °C inkubieren bis sich die Zellschicht löst (ca. 5-15min.). |
| | 10 ml Medium hinzufügen und Zellen lösen. |
| | Zentrifugieren für z.B. 7 Minuten bei 170g |
| | Überstand verwerfen und das Pellet in 10 ml Wachstumsmedium resuspendieren, Zellen zählen und mit Medium auf eine geeignete Dichte einstellen. |
| | Zellen werden bei 37 °C und 5 % $CO_2$ für ca. 3-5 Tage inkubiert und sollten 1-2 mal in der Woche passagiert werden. |
| Vorbereitung der Zellen für den Test | Zellen lösen wie oben beschrieben. |
| | Effektorzellen (MC 15) auf eine Konzentration von 2 x 10E6 Zellen/ml in Assaymedium einstellen. |
| | Zielzellen (C4-2) auf eine Konzentration von 4 x 10E5 Zellen/ml in Assaymedium einstellen. |
| | Mische gleiche Volumina von Ziel- und Effektorzellen 1:1 (Zellgemisch) |
| Vorbereitung der Proben, Assaykontrolle und Referenzmaterial | Äquilibriere Probe(n), Assaykontrolle und Referenzmaterial (RF) auf Raumtemperatur, gut mischen und alle auf die gleiche erste Konzentration (V1; z.B. 500 ng/mL) mit Assaymedium einstellen. Anschließend eine seriellen Verdünnung (z.B. 1:6, n = 7) mit Assaymedium durchführen um ausgeglichene Dosis-Wirkungs-Kurven zu erhalten. |
| Test Durchführung | |
| Zugabe der Proben, Assaykontrolle und Referenzmaterial | Überführe Aliquote von Proben, Assaykontrolle und Referenzmaterial (z.B. 25 μL) in die entsprechenden Kavitäten einer 96-well Flachbodenplatten. |
| | Überführe das Zellgemisch (z.B. 50 μl) in jede Kavität einer 96-well Flachbodenplatten. |
| | Platte schütteln z.B. 1 min mit 400 rpm |
| | Platte für 16 - 24 h bei 37 °C und 5 % $CO_2$ inkubieren |
| Bestimmung der Zytotoxizität und der relativen Aktivität | |
| Zugabe des Nachweisreagenzes und Messung | Verdünnung und Zugabe des Reagenzes sowie der nachfolgenden Messung werden in Übereinstimmung mit den Anweisungen des Herstellers z.B. CytoTox - Glo, Promega durchgeführt. |
| | 15 μL Reagenz pro Kavität hinzufügen |

(fortgesetzt)

Platte schütteln z.B. 1 min mit 400 rpm

ca. 15 min bei Raumtemperatur inkubieren

Die Lumineszenz wird mit einem geeigneten Multiplattenmessgerät gemessen.

Auswertung

| | |
|---|---|
| Ausgleichskurve | Bestimme die mittleren Messwerte bei jeder Konzentration für die Wiederholungen der Proben, Assaykontrolle und Referenzmaterial. |
| | Zeichne eine Dosis-Wirkungskurve für jede Serie der Proben, Assaykontrolle und Referenzmaterial. Hierzu werden die mittleren Messwerte über den finalen Konzentrationen von |
| | BAY2010112 aufgetragen (z.B. 500000 pg/ml bis 1.79 pg/ml). |
| | Lege eine geeignete Ausgleichskurve durch die mittleren Messwerte der Konzentrationsniveaus der Proben, Assaykontrolle und Referenzmaterial. |
| Relative Aktivität (Bestimmung) | Das Aktivitätsverhältnis zwischen Probe und Referenzmaterial wird bestimmt und dokumentiert. Statistische Software kann verwendet werden. |
| Beurteilung | Die relative Aktivität der Probe im Vergleich zum Referenzmaterial, muss der Spezifikation entsprechen. |

**Konzentrationsbestimmung der PSMA-BiTEl-Polypeptide (UV/VIS-Spektroskopie)**

Die Methode wird nach dem europäischem Arzneibuch durchgeführt (Ph. Eur., 2.2.25, UV-VIS-Spektroskopie bei 280 nm) und eignet sich auch für die Bestimmung der Konzentration anderer Moleküle.

Zunächst wurde experimentell der Extinktionskoeffizient der PSMA-BiTEl-Moleküle bestimmt. Dazu wurde die Extinktion von Lösungen mit bekannter PSMA-BiTEl-Konzentration bestimmt, wobei die Konzentrationen (in mol/l) anhand der molaren Masse der PSMA-BiTEl-Moleküle ($1,8673 \times 10^5$ g/mol) eingestellt wurden. Anhand der Extinktion, der Schichtdicke und der Konzentration ließ sich der Extinktionskoeffizient e von PSMA-BiTEl-Molekülen berechnen, gemäß

$$e = A / c \times d,$$

wobei

A = Extinktion (bzw. Absorption, unter Vernachlässigung der Lichtstreuung) bei einer geeigneten Wellenlänge (hier 280nm)

c = Konzentration (mol/l)

d = Schichtdicke (mm).

Die Extinktionswerte wurden dann auf der Ordinate gegen die zugehörigen Konzentrationen auf der Abszisse aufgetragen, um eine Eichgerade zu erhalten. Mittels dieser Eichgeraden konnte anschließend anhand der Extinktion die Konzentration PSMA-BiTE1-Lösung direkt abgelesen werden.

Für die Erstellung der genannten Eichgeraden muss das LAMBERT-BEER'sche Gesetz auf die gemessene Lösung anwendbar sein, d.h. u.a. die absorbierende Substanz muss homogen in der Lösung verteilt sein, die Variation des Absorptionskoeffizienten innerhalb des gemessenen Spektralbereiches muss vernachlässigbar sein und die gemessene Lösung muss hinreichend niedrig konzentriert sein, so dass keine wechselwirkungsbedingte Abweichungen auftreten.

Mithilfe des Extinktionskoeffizienten eines Moleküls lässt sich anhand der gemessenen Extinktion (bzw. Absorption bei 280 nm) umgekehrt auch die Proteinkonzentration (in mol/l) berechnen, gemäß:

$$\text{Proteinkonzentration [mol/l]} = A \times V / e \times d,$$

wobei

A = Absorption bei 280nm
d = Zellenlänge in cm (Standardzelle, 1.00 cm)
V = Verdünnung der Testlösung
e = Extinktionskoeffizient von PSMA-BiTE1 bei 280nm = 111315 $M^{-1} \times cm^{-1}$.

**Patentansprüche**

1. Eine flüssige pharmazeutische Zusammensetzung, umfassend ein Polypeptid, TRIS und Phosphat, wobei das Polypeptid die Aminosäuresequenz wiedergegeben in SEQ ID NO: 8 umfasst, **dadurch gekennzeichnet, dass** die Zusammensetzung das Polypeptid in einer Konzentration von 0,5 μg/mL bis 3.0 mg/ml, enthält, **dass** die Zusammensetzung 100 mM TRIS und 50 mM Phosphat enthält,

**dass** der pH-Wert der Zusammensetzung zwischen 5,0 und 7,5 liegt,
**dass** die Zusammensetzung 0,04% (m/V) Polysorbat 80 enthält und
**dass** die Zusammensetzung zusätzlich ein Lyoprotektivum enthält.

2. Eine Zusammensetzung nach Anspruch 1, **dadurch gekennzeichnet, dass** die Zusammensetzung 2 -10% (m/V) eines Lyoprotektivums enthält.

3. Eine Zusammensetzung nach einem der Ansprüche 2, **dadurch gekennzeichnet, dass** das Lyoprotektivum Trehalose oder Trehalose Dihydrat ist.

4. Eine Zusammensetzung nach einem der vorangehenden Ansprüche, **dadurch gekennzeichnet, dass** die Zusammensetzung das Polypeptid in einer Konzentration von 2mg/ml enthält.

5. Ein Feststoffgemisch, herstellbar durch Lyophilisation einer flüssigen Zusammensetzung nach einem der vorangehenden Ansprüche.

6. Eine flüssige pharmazeutische Zusammensetzung, **dadurch gekennzeichnet, dass** die Zusammensetzung durch Lösung eines lyophilisierten Feststoffgemisches nach Anspruch 5 in einem geeigneten flüssigen Medium rekonstituiert wird.

7. Eine Zusammensetzung nach einem der vorangehenden Ansprüche, **dadurch gekennzeichnet, dass** die Bioverfügbarkeit des Polypeptids nach subkutaner Gabe der Zusammensetzung >60% beträgt.

8. Eine Zusammensetzung nach einem der vorangehenden Ansprüche zur Anwendung in einem therapeutischen Verfahren.

9. Eine Zusammensetzung nach einem der vorangehenden Ansprüche zur Anwendung in einem therapeutischen Verfahren, wobei dieses Verfahren die parenterale Applikation der Zusammensetzung umfasst.

10. Eine Zusammensetzung nach einem der vorangehenden Ansprüche zur Verwendung in einem Verfahren zur therapeutischen Behandlung hyperproliferativer Erkrankungen der Prostata.

11. Eine Zusammensetzung nach einem der vorangehenden Ansprüche zur Verwendung in einem Verfahren zur therapeutischen Behandlung hyperproliferativer Erkrankungen der Prostata, wobei das Verfahren die subkutane Applikation der Zusammensetzung umfasst.

12. Spritze enthaltend eine Zusammensetzung nach einem der vorangehenden Ansprüche.

**Claims**

1. Liquid pharmaceutical composition comprising a polypeptide, TRIS and phosphate, the polypeptide comprising the amino acid sequence reproduced in SEQ ID NO: 8, **characterized in
that** the composition contains the polypeptide in a concentration of from 0.5 $\mu$g/ml to 3.0 mg/ml,
**that** the composition contains 100 mM TRIS and 50 mM phosphate,
**that** the pH of the composition is between 5.0 and 7.5,
**that** the composition contains 0.04% (m/v) polysorbate 80 and
**that** the composition additionally contains a lyoprotectant.

2. Composition according to Claim 1, **characterized in that** the composition contains 2 - 10% (m/v) of a lyoprotectant.

3. Composition according to any of Claims 2, **characterized in that** the lyoprotectant is trehalose or trehalose dihydrate.

4. Composition according to any of the preceding claims, **characterized in that** the composition contains the polypeptide in a concentration of 2 mg/ml.

5. Solids mixture obtainable by lyophilization of a liquid composition according to any of the preceding claims.

6. Liquid pharmaceutical composition, **characterized in that** the composition is reconstituted by dissolving a lyophilized solids mixture according to Claim 5 in a suitable liquid medium.

7. Composition according to any of the preceding claims, **characterized in that** the bioavailability of the polypeptide following subcutaneous administration of the composition is >60%.

8. Composition according to any of the preceding claims for use in a therapeutic method.

9. Composition according to any of the preceding claims for use in a therapeutic method, said method comprising parenteral administration of the composition.

10. Composition according to any of the preceding claims for use in a method for therapeutically treating hyper-proliferative diseases of the prostate.

11. Composition according to any of the preceding claims for use in a method for therapeutically treating hyper-proliferative diseases of the prostate, the method comprising subcutaneous administration of the composition.

12. Syringe containing a composition according to any of the preceding claims.

**Revendications**

1. Composition pharmaceutique liquide, comprenant un polypeptide, du TRIS et un phosphate, le polypeptide comprenant la séquence d'acides aminés indiquée dans SEQ ID NO : 8, **caractérisée en ce que**
la composition contient le polypeptide en une concentration de 0,5 $\mu$g/ml à 3,0 mg/ml,
la composition contient 100 mM de TRIS et 50 mM de phosphate,
le pH de la composition est compris entre 5,0 et 7,5,
la composition contient 0,04 % (m/V) de polysorbate 80 et
la composition contient en outre un lyoprotecteur.

2. Composition selon la revendication 1, **caractérisée en ce que** la composition contient 2 à 10 % (m/V) d'un lyoprotecteur.

3. Composition selon l'une quelconque de la revendication 2, **caractérisée en ce que** le lyoprotecteur est le tréhalose ou le dihydrate de tréhalose.

4. Composition selon l'une quelconque des revendications précédentes, **caractérisée en ce que** la composition contient le polypeptide en une concentration de 2 mg/ml.

5. Mélange solide, pouvant être fabriqué par lyophilisation d'une composition liquide selon l'une quelconque des revendications précédentes.

6. Composition pharmaceutique liquide, **caractérisée en ce que** la composition est reconstituée par dissolution d'un mélange solide lyophilisé selon la revendication 5 dans un milieu liquide approprié.

7. Composition selon l'une quelconque des revendications précédentes, **caractérisée en ce que** la biodisponibilité du polypeptide après administration sous-cutanée de la composition est > 60 %.

8. Composition selon l'une quelconque des revendications précédentes, destinée à une utilisation dans un procédé thérapeutique.

9. Composition selon l'une quelconque des revendications précédentes, destinée à une utilisation dans un procédé thérapeutique, ce procédé comprenant l'application parentérale de la composition.

10. Composition selon l'une quelconque des revendications précédentes, destinée à une utilisation dans un procédé de traitement thérapeutique de maladies hyperprolifératives de la prostate.

11. Composition selon l'une quelconque des revendications précédentes, destinée à une utilisation dans un procédé de traitement thérapeutique de maladies hyperprolifératives de la prostate, le procédé comprenant l'application sous-cutanée de la composition.

12. Seringue contenant une composition selon l'une quelconque des revendications précédentes.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2011061712 A
- WO 2010148337 A1
- WO 2009070642 A1
- WO 2008119566 A2
- WO 2008119567 A2
- WO 2010037836 A2

**In der Beschreibung aufgeführte Nicht-Patentliteratur**

- **BÄUERLE et al.** *Curr Opin Mol Ther.,* Februar 2009, vol. 11 (1), 22-30
- Remington's Pharmaceutical Sciences. Mack Publishing Co,